(19) Europäisches Patentamt
European Patent Office
Office européen des brevets

(11) EP 1 652 926 B1

(12) **EUROPEAN PATENT SPECIFICATION**

(45) Date of publication and mention of the grant of the patent:
**30.09.2009 Bulletin 2009/40**

(21) Application number: **05023081.2**

(22) Date of filing: **26.04.2001**

(51) Int Cl.:
*C12N 15/52* (2006.01) *C12N 1/21* (2006.01)
*C12P 17/16* (2006.01) *C07D 313/00* (2006.01)
*C07D 413/06* (2006.01) *C07D 417/06* (2006.01)
*C12R 1/01* (2006.01)

(54) **CRYSTALLINE EPOTHILONE D**

KRISTALLINES EPOTHILON D

EPOTHILONE D CRISTALLINE

(84) Designated Contracting States:
**AT BE CH CY DE DK ES FI FR GB GR IE IT LI LU MC NL PT SE TR**

(30) Priority:
**28.04.2000 US 560367**
**14.09.2000 US 232696 P**
**21.12.2000 US 257517 P**
**03.04.2001 US 825856**
**03.04.2001 US 825876**
**13.02.2001 US 269020 P**

(43) Date of publication of application:
**03.05.2006 Bulletin 2006/18**

(62) Document number(s) of the earlier application(s) in accordance with Art. 76 EPC:
**01973782.4 / 1 320 611**

(73) Proprietor: **Kosan Biosciences, Inc.**
**Hayward, CA 94545 (US)**

(72) Inventors:
- **Arslanian, Robert L.**
  **Pacifica, CA 94044 (US)**
- **Ashley, Gary**
  **Alameda, CA 94502 (US)**
- **Frykman, Scott**
  **San Francisco, CA 94109-2026 (US)**
- **Julien, Bryan**
  **Oakland, CA 94619 (US)**
- **Katz, Leonard**
  **Oakland, CA 94611 (US)**
- **Khosla, Chaitan**
  **Palo Alto, CA 94306 (US)**
- **Lau, Janice**
  **San Mateo, CA 94403 (US)**
- **Licari, Peter J.**
  **Fremont, CA 94536 (US)**
- **Regentin, Rika**
  **Hayward, CA 94542 (US)**
- **Santi, Daniel**
  **San Francisco, CA 94117 (US)**
- **Tang, Li**
  **Foster City, CA 94404 (US)**

(74) Representative: **Roques, Sarah Elizabeth**
**J.A. Kemp & Co.**
**14 South Square**
**Gray's Inn**
**London WC1R 5JJ (GB)**

(56) References cited:
**WO-A-00/22139** **WO-A-00/31247**
**WO-A-98/22461** **WO-A-99/42602**
**WO-A-99/66028**

- **TANG L ET AL.: "Cloning and heterologous expression of the epothilone gene cluster" SCIENCE, vol. 287, no. 5453, 2000, pages 640-642, XP000910114 ISSN: 0036-8075**
- **BEYER S ET AL.: "Metabolic diversity in myxobacteria: identification of the myxalamid and the stigmatellin biosynthetic gene cluster of Stigmatella aurantiaca Sg a15 and a combined polyketide-(poly)peptide gene cluster from the epothilone producing strain Sorangium cellulosum So ce90" BIOCHIMICA ET BIOPHYSICA ACTA. GENE STRUCTURE AND EXPRESSION, vol. 1445, no. 2, 14 May 1999 (1999-05-14), pages 185-195, XP004275376 ISSN: 0167-4781**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 652 926 B1

- SILAKOWSKI B ET AL.: "New lessons for combinatorial biosynthesis from myxobacteria: The myxothiazol biosynthetic gene cluster of Stigmatella aurantiaca DW4/3-1." JOURNAL OF BIOLOGICAL CHEMISTRY, vol. 274, no. 52, 24 December 1999 (1999-12-24), pages 37391-37399, XP002199229 ISSN: 0021-9258
- PAITAN Y ET AL.: "The first gene in the biosynthesis of the polyketide antibiotic TA of Myxococcus xanthus codes for a unique PKS module coupled to a peptide synthetase" JOURNAL OF MOLECULAR BIOLOGY, LONDON, GB, vol. 286, no. 1, 1999, pages 465-474, XP000920956 ISSN: 0022-2836
- SHIMKETS L J: "Industrial relevance and genetic analysis of myxobacteria." INDUSTRIAL MICROORGANISMS: BASIC AND APPLIED MOLECULAR GENETICS., 1993, pages 85-96, XP008003384 Fifth ASM;Bloomington, Indiana, USA; October 11-15, 1992, American Society for Microbiology (ASM) Books Division, 1325 Massachusetts Ave. NW, Washington, DC 20005-4171, USA ISBN: 1-55581-063-2
- NICOLAOU K C ET AL.: "Chemical Biology of Epothilones" ANGEWANDTE CHEMIE INTERNATIONAL EDITION, vol. 37, no. 15, August 1998 (1998-08), pages 2015-2045, XP002131418 ISSN: 0570-0833
- BRETSCHER A P ET AL.: "Nutrition of Myxococcus xanthus, a fruiting Myxobacterium" JOURNAL OF BACTERIOLOGY, vol. 133, no. 2, 1978, pages 763-768, XP001010255 ISSN: 0021-9193
- HÖFLE ET AL: "Epothilone A and B-Novel 16-membered macrolides with cytotoxic activity: Isolation, crystal structure and conformation in solution" ANGEWANDTE CHEMIE. INTERNATIONAL EDITION, vol. 35, no. 13/14, 1996, pages 1567-1569, XP002111284 ISSN: 1433-7851
- REGENTIN R ET AL.: "Development of a cost effective epothilone D process in Myxococcus xanthus." ABSTRACTS OF PAPERS AMERICAN CHEMICAL SOCIETY, vol. 221, no. 1-2, 1 April 2001 (2001-04-01), page BIOT 61, XP008003385 221st National Meeting of the American Chemical Society;San Diego, California, USA; April 01-05, 2001 ISSN: 0065-7727
- PFEIFER B A ET AL.: "Biosynthesis of polyketides in heterologous hosts" MICROBIOLOGY AND MOLECULAR BIOLOGY REVIEWS, vol. 65, no. 1, March 2001 (2001-03), pages 106-118, XP000997626 ISSN: 1092-2172
- FRYKMAN S ET AL.: "Modulation of epothilone analog production through media design." JOURNAL OF INDUSTRIAL MICROBIOLOGY & BIOTECHNOLOGY, vol. 28, no. 1, January 2002 (2002-01), pages 17-20, XP008003393 ISSN: 1367-5435
- ARSLANIAN R L ET AL.: "Large-scale isolation and crystallization of epothilone D from Myxococcus xanthus cultures." JOURNAL OF NATURAL PRODUCTS, vol. 65, no. 4, April 2002 (2002-04), pages 570-572, XP001074701 ISSN: 0163-3864
- LAU J ET AL.: "Optimizing the heterologous production of epothilone D in Myxococcus xanthus." BIOTECHNOLOGY AND BIOENGINEERING, vol. 78, no. 3, 5 May 2002 (2002-05-05), pages 280-288, XP002199234 ISSN: 0006-3592

## Description

### Field of the Invention

The present invention provides crystalline forms of epothilone D and methods for obtaining the crystalline forms. The invention relates to the fields of agriculture, chemistry, medicinal chemistry, medicine, molecular biology, and pharmacology.

### Background of the Invention

Polyketides constitute a class of structurally diverse compounds synthesized, at least in part, from two carbon unit building block compounds through a series of Claisen type condensations and subsequent modifications. Polyketides include antibiotics such as tetracycline and erythromycin, anticancer agents such as the epothilones and daunomycin, and immunosuppressants such as FK506, FK520, and rapamycin. Polyketides occur naturally in many types of organisms, including fungi and mycelial bacteria. Polyketides are synthesized *in vivo* by polyketide synthase enzymes commonly referred to as PKS enzymes. Two major types of PKS are known that differ in their structure and the manner in which they synthesize polyketides. These two types are commonly referred to as Type I or modular and Type II or iterative (aromatic) PKS enzymes.

Modular PKS enzymes are typically multi-profiein complexes in which each protein contains multiple active sites, each of which is used only once during carbon chain assembly and modification. Iterative PKS enzymes are typically multi-protein complexes in which each protein contains only one or at most two active sites, each of which is used multiple times during carbon chain assembly and modification. A large number of the genes for both modular and aromatic PKS enzymes have been cloned.

Modular PKS genes are composed of coding sequences organized to encode a loading module, a number of extender modules, and a releasing domain. As described more fully below, each of these domains and modules corresponds to a polypeptide with one or more specific functions. Generally, the loading module is responsible for binding the first building block used to synthesize the polyketide and transferring it to the first extender module. The building blocks used to form complex polyketides are typically acylthioesters, most commonly acetyl, propionyl, malonyl, methylmalonyl, hydroxymalonyl, methoxymalonyl, and ethylmalonyl CoA. Other building blocks include amino acid and amino acid-like acylthioesters. PKSs catalyze the biosynthesis of polyketides through repeated, decarboxylative Claisen condensations between the acylthioester building blocks. Each module is responsible for binding a building block, performing one or more functions on that building block, and transferring the resulting compound to the next module. The next module, in turn, is responsible for attaching the next building block and transferring the growing compound to the next module until synthesis is complete. At that point, the releasing domain, often an enzymatic thioesterase (TE) activity, cleaves the polyketide from the PKS.

Recombinant methods for manipulating modular and iterative PKS genes are described in U.S. Patent Nos. 5,962,290; 5,672,491; 5,712,146; 5,830,750; and 5,843,718; and in PCT patent publication Nos. 98/49315 and 97/02358,. These and other patents describe recombinant expression vectors for the heterologous production of polyketides as well as recombinant PKS genes assembled by combining parts of two or more different PKS genes or gene clusters that produce novel polyketides. To date, such methods have been used to produce known or novel polyketides in organisms such as *Streptomyces*, which naturally produce polyketides, and E. coli and yeast, which do not naturally produce polyketides (see U.S. Patent No. 6,033,883, ). In the latter hosts, polyketide production is dependent on the heterologous expression of a phosphopantetheinyl transferase, which activates the ACP domains of the PKS (see PCT publication No. 97/13845, ).

While such methods are valuable and highly useful, certain polyketides are expressed only at very low levels in, or are toxic to, the heterologous host cell employed. As an example, the anticancer agents epothilones A, B, C, and D were produced in *Streptomyces* by heterologous expression of the epothilone PKS genes (Tang et al., 28 Jan. 00, Clowning and heterologous expression of the epothilone gene cluster, Science, 287: 640-642, and PCT Pub. No. 00/031247, ). Epothilones A and B were produced at less than about 50 to 100 μg/L and appeared to have a deleterious effect on the producer cells.

Epothilones A and B were first identified as an antifungal activity extracted from the myxobacterium *Sorangium cellulosum* (see Gerth et al., 1996, J. Antibiotics 49: 560-563 and Germany Patent No. DE 4138 042, ) and later found to have activity in a tubulin polymerization assay (see Bollag et al., 1995, Cancer Res. 55:2325-2333, ). Epothilones A and B and certain naturally occurring and synthetic derivatives have since been extensively studied as potential antitumor agents for the treatment of cancer. The chemical structures of the epothilones produced by *Sorangium cellulosum* strain So ce 90 were described in Hofle et al., 1996, Epothilone A and B - novel 16-membered macrolides with cytotoxic activity: isolation, crystal structure, and conformation in solution, Angew. Crem. Int. Ed. Engl. 35(13/14): 1567-1569, Epothilones A (R = H) and B (R = $CH_3$) have the structure shown below and show broad cytotoxic activity against eukaryotic cells

and noticeable activity and selectivity against breast and colon tumor cell lines.

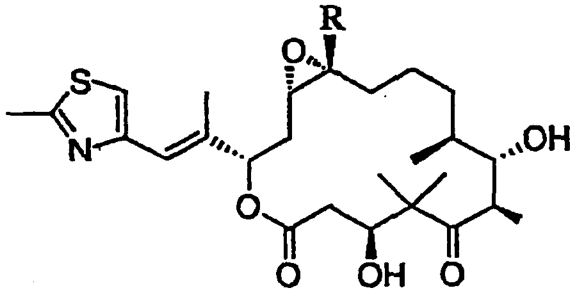

The desoxy counterparts of epothilones A and B, also known as epothilones C (R = H) and D (R = $CH_3$), have been chemically synthesized *de novo* but are also present as minor products in fermentations of *S. cellulosum*. Epothilones C and D are less cytotoxic than epothilones A and B; their structures are shown below.

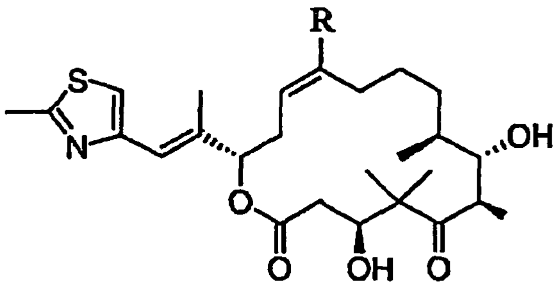

Other naturally occurring epothilones have been described. These include epothilones E and F, in which the methyl side chain of the thiazole moiety of epothilones A and B has been hydroxylated to yield epothilones E and F, respectively, as well as many other epothilone compounds (see PCT Pub. No. 99/65913,).

Because of the potential for use of the epothilones as anticancer agents, and because of the low levels of epothilone produced by the native So ce 90 strain, a number of research teams undertook the effort to synthesize the epothilones. As noted above, this effort has been successful (see Balog et al., 1996, Total synthesis of (-)-epothilone A, Angew. Chem. Int. Ed. Engl. 35(23/24): 2801-2803; Su et al., 1997, Total synthesis of (-)-epothilone B: an extension of the Suzuki coupling method and insights into structure-activity relationships of the epothilones, Angew. Chem. Int. Ed. Engl. 36(7): 757-759; Meng et al., 1997, Total syntheses of epothilones A and B, JACS 119(42): 10073-10092; and Balog et al., 1998, A novel aldol condensation with 2-methyl-4-pentenal and its application to an improved total synthesis of epothilone B, Angew. Chem. Int. Ed. Engl. 37(19): 2675-2678 ). Despite the success of these efforts, the chemical synthesis of the epothilones is tedious, time-consuming, and expensive. Indeed, the methods have been characterized as impractical for the full-scale pharmaceutical development of any epothilone as an anticancer agent.

A number of epothilone derivatives, as well as epothilones A - D, have been studied *in vitro* and *in vivo* (see Su et al., 1997, Structure-activity relationships of the epothilones and the first in vivo comparison with paclitaxel, Angew. Chem. Int. Ed. Engl. 36(19): 2093-2096; and Chou et al., Aug. 1998, Desoxyepothilone B: an efficacious microtubule-targeted antitumor agent with a promising in vivo profile relative to epothilone B, Proc. Natl. Acad. Sci. USA 95: 9642-9647, ). Additional epothilone derivatives and methods for synthesizing epothilones and epothilone derivatives are described in PCT Pub. Nos. 00/23452, 00/00485, 99/67253, 99/67252, 99/65913, 99/54330, 99/54319, 99/54318, 99/43653, 99/43320, 99/42602, 99/40047, 99/27890, 99/07692, 99/02514, 99/01124, 98/25929, 98/22461, 98/08849, and 97/19086; U.S. Patent No. 5,969,145; and Germany patent publication No. DE 4138 042,

There remains a need for economical means to produce not only the naturally occurring epothilones but also the derivatives or precursors thereof, as well as new epothilone derivatives with improved properties.

## Summary of the Invention

The present invention relates to methods for purifying an epothilone. The epothilone may be purified from

fermentation broth.

An epothilone compound may be provided in a highly purified form. The epothilone may be more than 95% pure. The epothilone may be more than 99% pure. The epothilone may be in crystalline form.

The present invention provides crystalline epothilone D. The present invention also provides a method for obtaining epothilone D in crystalline form comprising crystallizing epothilone D from a binary solvent system in which water is the forcing solvent. Preferably the binary solvent system is ethanol and water and in this embodiment the epothilone D in crystalline form may be more than 95% pure.

A method of treating cancer, may comprise administering a therapeutically effective amount of an epothilone compound as described herein. The compounds and compositions described herein are also useful in the treatment of other hyperproliferative diseases and conditions, including, but not limited to, psoriasis and inflammation

These and other embodiments of the invention are described in more detail in the following description, the examples, and claims set forth below.

## Brief Description of the Figures

Figure 1 shows restriction site and function maps of plasmids pK0S35-821 and pK0S35-822.

Figure 2 shows restriction site and function maps of plasmids pKOS35-154 and pKOS90-22.

Figure 3 shows a schematic of a protocol for introducing the epothilone PKS and modification enzyme genes into the chromosome of a *Myxococcus xanthus* host cell as described in Example 2.

Figure 4 shows a map of pBeloBACII as described in Example 2.

Figure 5 shows the baseline performance of *Myxococcus xanthus* strain K111-40-1 in a simple production medium consisting only of 5 g/L casitone (a pancreatic casein digest) and 2 g/L magnesium sulfate. **Legend**: Growth (•), production (■), and ammonia generation (▲) profiles for the basal CTS medium in a batch process; culture conditions were as described in Materials and Methods in Example 3.

Figure 6 shows the effect of XAD-16 resin on the fermentation performance of *Myxococcus xanthus* strain K111-40-1 in CTS production medium. **Legend**: Growth (•) and production (■) profiles with the incorporation of 20 g/L XAD-16 resin to the CTS production medium in a batch process.

Figure 7 shows the influence of casitone on growth and product yield. Legend: Effect of casitone concentration on growth (•), production (■), and specific productivity (▲).

Figure 8 shows the influence of trace elements and higher methyl oleate concentrations on growth and product yield. Legend: Effect of methyl oleate (• and trace elements (■) on production.

Figure 9A shows the growth and production of the *M. xanthus* strain in the presence of optimal concentrations of methyl oleate and trace elements in a batch fermentation process. Exponential growth of the occurred during the first two days after inoculation. Production of epothilone D began at the onset of the stationary phase and ceased when cell lysis occurred with the depletion of methyl oleate on day 5. Figure 9B shows the time courses corresponding to the consumption of methyl oleate and generation of ammoniaduring the growth and proudction of the *M. xanthus* strain in the presence of optimal concentrations of methyl oleate and trace elements in a batch fermentation process. Legend: A.) Growth (•) and production (■) profiles with the addition of optimal concentrations of methyl oleate (7 mL/L) and trace elements (4 mL/L) to the CTS production medium in a batch process. B.) Time courses corresponding to the consumption of methyl oleate (•) and the generation of ammonia (■).

Figure 10A shows the influence of intermittent fed-batch process on the growth and production of the *M. xanthus* strain. Legend: Growth (•) and production (■) profiles for the intermittent fed-batch process in shake-flasks. The casitone and methyl oleate feed rates were 2 g/L/day and 3 mL/L/day, respectively. Figure 10B shows the constant rates of consumption of methyl oleate and generation of ammonia during the course of the fermentation. **Legend**: Time courses corresponding to the total addition of methyl oleate to the cultures (•), the total consumption of methyl oleate (■), and the generation of ammonia (▲).

Figure 11 shows the production profile for the intermittent fed-batch process in a 5-L bioreactor. The casitone and methyl oleate feed rates were 2 g/L/day and 3 mL/L/ day, respectively.

Figure 12A shows the impact of continous feeds on growth and production. Legend: Growth (•) and production (■) profiles for the continuous fed-batch process in a 5-L bioreactor. The casitone and methyl oleate feed rates were 2 g/L/day and 3 mL/L/day, respectively. Figure 12B shows the time course of methyl oleate addition and consumption as well as the generation of ammonia during the continuous fed-batch process. Legend: Time courses corresponding to the total addition of methyl oleate to the cultures (•), the total consumption of methyl oleate (■), and the generation of ammonia (▲); culture conditions were as described in Materials and Methods

## Detailed Description of the Invention

Statements regarding the scope of the present invention and definitions of terms used herein are listed below.

The definitions apply to the terms as they are used throughout this specification, unless otherwise limited in specific instances, either individually or as part of a larger group.

All stereoisomers of the compound described herein are included within the scope of the invention, as pure compounds as well as mixtures thereof. Individual enantiomers, diastereomers, geometric isomers, and combinations and mixtures thereof are all encompassed by the present invention. Furthermore, some of the crystalline forms for the compounds may exist as polymorphs and as such are included in the present invention. In addition, some of the compounds may form solvates with water (i.e., hydrates) or common organic solvents, and such solvates are also encompassed within the scope of this invention.

Protected forms of the inventive compounds are included within the scope of the present invention. A variety of protecting groups are disclosed, for example, in T. H. Greene and P.G. M. Wuts, Protective Groups in Organic Synthesis, Third Edition, John Wiley & Sons, New York (1999), which is incorporated herein by reference in its entirety. For example, a hydroxy protected form of the inventive compounds are those where at least one of the hydroxyl groups is protected by a hydroxy protecting group. Illustrative hydroxyl protecting groups include but not limited to tetrahydropyranyl; benzyl; methylthiomethyl; ethylthiomethyl; pivaloyl; : phenylsulfonyl; triphenylmethyl, trisubstituted silyl such as trimethyl silyl, triethylsilyl, tributylsilyl, tri-isopropylsilyl, t-butyldimethylsilyl, tri-t-butylsilyl, methyldiphenylsilyl, ethyldiphenylsilyl, t-butyldiphenylsilyl and the like; acyl and aroyl such as acetyl, pivaloylbenzoyl, 4-methoxybenzoyl, 4-nitrobenzoyl and aliphatic acylaryl and the like. Keto groups in the inventive compounds may similarly be protected.

The term "isolated" as used herein to refer to a compound means altered "by human intervention from its natural state. For example, if the compound occurs in nature, it has been changed or removed from its original environment, or both. In other words, a compound naturally present in a living organism is not "isolated," but the same compound separated from the coexisting materials of its natural state is "isolated", as the term is employed herein. The term "isolated" can also mean a compound that is in a preparation that is substantially free of contaminating or undesired materials. With respect to compounds found in nature, substantially free of the materials with which that compound or composition is associated in its natural state.

The term "purified" as it refers to a compound means that the compound is in a preparation in which the compound forms a major component of the preparation, such as constituting about 50%, about 60%, about 70%, about 80%, about 90%, about 95% or more by weight of the components in the preparation.

The term "subject" as used herein, refers to an animal, preferably as mammal, who has been the object of treatment, observation or experiment and most preferably a human who has been the object of treatment and/or observation.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

The term "composition" is intended to encompass a product comprising the specified ingredients in the specified amounts, as well as any product that results, directly or indirectly, from combinations of the specified ingredients in the specified amounts.

The present invention provides crystalline forms of epothilone D.

Recombinant host cells of the suborder *Cystobacterineae* containing recombinant expression vectors that encode heterologous PKS genes may be used to produce polyketides synthesized by the PKS enzymes encoded on those vectors. As used herein, the term recombinant refers to a cell, compound, or composition produced by human intervention, typically by specific and directed manipulation of a gene or portion thereof. The suborder *Cystobacterineae* is one of two (the other is *Sorangineae*, which includes the epothilone producer *Sorangium cellulosum*) in the order *Myxococcales*. The suborder *Cystobacterineae* includes the family *Myxococcaceae* and the family *Cystobacteraceae*. The family *Myxococcacceae* includes the genus *Angiococcus* (i.e, *A. disciformis*), the genus *Myxococcus*, and the genus *Corallococcus* (i.e., *C. macrosporus*, *C. corralloides*, and *C. exiguus*). The family *Cystobacteraceae* includes the genus *Cystobacter* (i.e., *C. fuscus*, *C. ferrugineus*, *C. minor*, *C. velatus*, and *C. violaceus*), the genus *Melittangium* (i.e., *M. boletus* and *M. lichenicola*), the genus *Stigmatella* (i.e., *S. erecta* and *S. aurantiaca*), and the genus *Ardiangium* (i.e., *A. gephyra*). Especially preferred host cells are those that produce a polyketide at equal to or greater than. 10 to 20 mg/L, more preferably at equal to or greater than 100 to 200 mg/L, and most preferably at equal to or greater than 1 to 2 g/L.

The host cells may be from the genus *Myxococcus* or the genus *Stigmatella.* The host cell may be selected from the group consisting of *M. stipitatus, M. fulous, M. xanthus*, *M. virescens, S. erecta,* and *S. aurantiaca*. Especially preferred *Myxococcus* host cells are those that produce a polyketide at equal to or greater than 10 to 20 mg/L, more preferably at equal to or greater than 100 to 200 mg/L, and most preferably at equal to or greater than 1 to 2 g/L. Especially preferred are *M. xanthus* host cells that produce at these levels. *M. xanthus* host cells that can be employed include, but are not limited to, the DZ1 cell line (Campos et al., 1978, J. Mol. Biol. 119:167-178,), the TA-producing cell line ATCC 31046, the DK1219 cell line (Hodgkin and Kaiser, 1979, Mol. Gen. Genet. 171: 177-191,), and the DK1622 cell line (Kaiser, 1979, Proc. Natl. Acad. Sci. USA 76:5952-5956, ).

Host cells as described herein comprise a recombinant DNA expression vector. Recombinant DNA vectors may be capable of chromosomal integration or extrachromosomal replication in these host cells. The vectors comprise at least a portion of a PKS coding sequence and are capable of directing expression of a functional PKS enzyme in the host cells. As used herein, the term expression vector refers to any nucleic acid that can be introduced into a host cell. An expression vector can be maintained stably or transiently in a cell, whether as part of the chromosomal or other DNA in the cell or in any cellular compartment, such as a replicating vector in the cytoplasm. An expression vector also comprises a gene that serves to direct the synthesis of RNA that is translated into a polypeptide in the cell or cell extract. Thus, the vector either includes a promoter to enhance gene expression or is integrated into a site in the chromosome such that gene expression is obtained. Furthermore, expression vectors typically contain additional functional elements, such as resistance-conferring genes to act as selectable markers and regulatory genes to enhance promoter activity.

Typically, the expression vector will comprise one or more marker genes by which host cells containing the vector can be identified and/or selected. Illustrative antibiotic resistance conferring genes for use in vectors include the *ermE* (confers resistance to erythromycin and lincomycin), tsr (confers resistance to thiostrepton), *aadA* (confers resistance to spectinomycin and streptomycin), *aacC4* (confers resistance to apramycin, kanamycin, gentamicin, geneticin (G418), and neomycin), *hyg* (confers resistance to hygromycin), and *vph* (confers resistance to viomycin) resistance conferring genes. Selectable markers for use in *Myxococcus xanthus* include kanamycin, tetracycline, chloramphenicol, zeocin, spectinomycin, and streptomycin resistance conferring genes.

The various components of an expression vector can vary widely, depending on the intended use of the vector. In particular, the components depend on the host cell(s) in which the vector will be used and the manner in which it is intended to function. For example, certain preferred vectors of the invention are integrating vectors: the vectors integrate into the chromosomal DNA of the host cell. Such vectors can comprise a phage attachment site or DNA segments complementary to segments of the host cell chromosomal DNA to direct integration. Moreover, a series of such vectors can be used to build the PKS gene cluster in the host cell, with each vector comprising only a portion of the complete PKS gene cluster. Thus, the recombinant DNA expression vectors may comprise only a portion of a PKS gene or gene cluster. Homologous recombination can also be used to delete, disrupt, or alter a gene, including a heterologous PKS gene previously introduced into the host cell.

A wide variety of promoters are available for use in the preferred *Myxococcus* expression vectors. For example, the promoter of the *Sorangium cellulosum* epothilone PKS gene (see PCT Pub. No. 00/031247,) functions in *M. xanthus* host cells. The epothilone PKS gene promoter can be used to drive expression of one or more epothilone PKS genes or another PKS gene product in recombinant host cells. Another preferred promoter for use in *M. xanthus* host cells for purposes of expressing a recombinant PKS is the promoter of the *pilA* gene of *M. xanthus*. This promoter, as well as two *M. xanthus* strains that express high levels of gene products from genes controlled by the *pilA* promoter, a *pilA* deletion strain and a *pilS* deletion strain, are described in Wu and Kaiser, Dec. 1997, Regulation of expression of the pilA gene in Myxococcus xanthus, J. Bact. 179(24):7748-7758, Recombinant *Myxococcus* host cells may comprise both the *pilA* and *pilS* deletions. Another preferred promoter is the starvation dependent promoter of the *sdeK* gene.

Preferred expression vectors for use in preparing the recombinant *Myxococcus xanthus* expression vectors and host cells are designated plasmids pKOS35-82.1 and pKOS35-822 (Figure 1), are able to replicate in *E. coli* host cells as well as integrate into the chromosomal DNA of *M. xanthus*. The vectors comprise the Mx8 attachment and integration genes as well as the *pilA* promoter with restriction enzyme recognition sites placed conveniently downstream. The two vectors differ from one another merely in the orientation of the *pilA* promoter on the vector and can be readily modified to include the epothilone PKS and modification enzyme genes of the invention or other PKS and modification enzyme genes. The construction of the vectors is described in Example 1.

A method for producing a polyketide in a host cell of the suborder *Cystobacterineae*, which polyketide is not naturally produced in said host cell, may comprise culturing the host cell transformed with a recombinant DNA vector under conditions such that a PKS gene encoded on the vector is expressed and said polyketide is produced. With this method, any of the diverse members of the polyketides produced by modular or iterative PKS enzymes can be prepared. In addition, novel polyketides derived from hybrid or other recombinant PKS genes can also be prepared using this method.

A large number of modular PKS genes have been cloned and are immediately available for use in the vectors and methods described herein. The polyketides produced by PKS enzymes are often further modified by polyketide modification enzymes, also called tailoring enzymes, that hydroxylate, epoxidate, methylate, and/or glycosylate the polyketide product of the PKS. These genes can also be introduced into the host cell to prepare a modified polyketide of interest.

To construct any PKS described herein, one can employ a technique, described in PCT Pub. No. 98/27203 and U.S. Patent No. 6,033,883, in which the various genes of the PKS and optionally genes for one or more polyketide modification enzymes are divided into two or more, often three, segments, and each segment is placed on a separate expression vector (see also PCT Pub. No. 00/063361,). In this manner, the full complement of genes can be assembled and manipulated more readily for heterologous expression, and each of the segments of the gene can be altered, and various altered segments can be combined in a single host cell to provide a recombinant PKS gene of the invention.

This technique makes more efficient the construction of large libraries of recombinant PKS genes, vectors for expressing those genes, and host cells comprising those vectors. In this and other contexts, the genes encoding the desired PKS not only can be present on two or more vectors, but also can be ordered or arranged differently from that which exists in the native producer organism from which the genes were derived.

The recombinant host cell may produce epothilone or an epothilone derivative. The naturally occurring epothilones (including epothilone A, B, C, D, E, and F) and non-naturally occurring compounds structurally related thereto (epothilone derivatives or analogs) are potent cytotoxic agents specific for eukaryotic cells. These compounds have application as anti-fungals, cancer chemotherapeutics, and immunosuppressants, and generally for the treatment of inflammation or any hyperproliferative disease, such as psoriasis, multiple sclerosis, atherosclerosis, and blockage of stents. The epothilones are produced at very low levels in the naturally occurring *Sorangium cellulosum* cells in which they have been identified.

The host cells described herein may produce less complex mixtures of epothilones than do naturally occurring cells that produce epothilones. As one example, certain host cells can produce epothilone D in a less complex mixture than is produced by a naturally occurring *Sorangium cellulosum*, because epothilone D is a major product in the former and a minor product in the latter. Naturally occurring *Sorangium cellulosum* cells that produce epothilones typically produce a mixture of epothilones A, B, C, D, E, F, and other very minor products, with only epothilones A and B present as major products. The Table 1 below summarizes the epothilones produced in different illustratrive host cells described herein.

**TABLE 1**

| Cell Type | Epothilones Produced | Epothilones Not Produced* |
|---|---|---|
| 1 | A, B, C, D | E, F |
| 2 | A, C | B, D, E, F |
| 3 | B,D | A, C, E, F |
| 4 | A, B | C,D |
| 5 | C, D | A, B |
| 6 | B | A, C, D, E, F |
| 7 | D | A, B, C, E, F |
| *or produced only as minor products | | |

Thus, the recombinant host cells may produce as a major product only one desired epothilone or epothilone derivative.

Based solely on an analysis of the domains of the epothilone PKS, one could predict that the PKS enzyme catalyzes the production of epothilones arbitrarily designated "G" and "H", the structures of which are shown below:

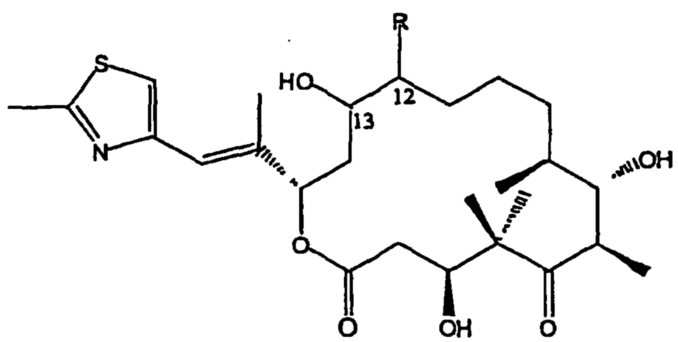

Epothilones G (R=H) and H (R=$CH_3$).

These compounds differ from one another in that epothilone G has a hydrogen at C-12 and epothilone H has a methyl group at that position. The variance at the C-12 position is predicted to arise from the ability of the corresponding

AT domain (extender module 4) of the PKS to bind either malonyl CoA, leading to hydrogen, or methylmalonyl CoA, leading to methyl. However, epothilones G and H have not been observed in nature or in the recombinant host cells described herein. Instead, the products of the PKS are believed to be epothilones C and D, which differ from epothilones G and H, respectively, by having a C-12 to C-13 double bond and lacking a C-13 hydroxyl substituent Based on the expression of the epothilone PKS genes in heterologous host cells and the products produced by genetic alteration of those genes, as described more fully below, the dehydration reaction that forms the C12-C13 double bond in epothilones C and D is believed to be carried out by the epothilone PKS itself. Epothilones A and B are formed from epothilones C and D, respectively, by epoxidation of the C-12 to C-13 double bond by the *epoK* gene product. Epothilones E and F may be formed from epothilones A and B, respectively, by hydroxylation of the C-21 methyl group or by incorporation of a hydroxymalonyl CoA instead of a malonyl CoA by the loading module of the epothilone PKS, as discussed further below.

Thus expression of the epothilone PKS genes and the *epoK* gene in a host cell of the invention leads to the production of epothilones A, B, C, and D. If the *epoK* gene is not present or is rendered inactive or partially inactive by mutation, then epothilones C and D are produced as major products. If the AT domain of extender module 4 is replaced by an AT domain specific for malonyl CoA, then epothilones A and C are produced, and if there is no functional *epoK* gene, then epothilone C is produced as the major product If the AT domain of extender module 4 is replaced by an AT domain specific for methylmalonyl Co A, then epothilones B and D are produced as major products, and if there is no functional *epoK* gene, then epothilone D is produced as the major product

The epothilone PKS and modification enzyme genes were cloned from the epothilone producing strain, *Sorangium cellulosum* SMP44. Total DNA was prepared from this strain using the procedure described by Jaoua et al., 1992, Plasmid 28: 157-165,. A cosmid library was prepared from *S. cellulosum* genomic DNA in pSupercos (Stratagene). The entire PKS and modification enzyme gene cluster was isolated in four overlapping cosmid clones (deposited on February 17,1999, under the terms of the Budapest Treaty with the American Type Culture Collection (ATCC), 10801 University Blvd., Manassas, VA, 20110-2209 USA, and assigned ATCC accession numbers as follows: pKOS35-70.1A2 (ATCC 203782), pKOS35-70.4 (ATCC 203781), pKOS35-70.8A3 (ATCC 203783), and pKOS35-79.85 (ATCC 203780)) and the DNA sequence determined, as described in PCT Pub. No. 00/031237,. DNA sequence analysis revealed a PKS gene cluster with a loading module and nine extender modules. Downstream of the PKS sequence is an open reading frame (ORF), designated *epoK*, that shows strong homology to cytochrome P450 oxidase genes and encodes the epothilone epoxidase modification enzyme.

The PKS genes are organized in 6 ORFs. At the polypeptide level, the loading module and extender modules 1 (an NRPS), 2, and 9 appear on individual polypeptides; their corresponding genes are designated *epoA, epoB, epoC*, and *epoF* respectively. Modules 3, 4, 5, and 6 are contained on a single polypetide whose gene is designated *epoD*, and modules 7 and 8 are on another polypeptide whose gene is designated *epoE*. The spacing between ORFs suggests that *epoC, epoD, epoE* and *epoF* constitute an operon. The *epoA, epoB*, and *epoK* gene may be also part of this large operon, but there are spaces of approximately 100 bp between *epoB* and *epo*C and 115 bp between *epoF* and *epoK* that could contain a promoter. The epothilone PKS gene cluster is shown schematically below in Scheme 1.

**SCHEME 1**

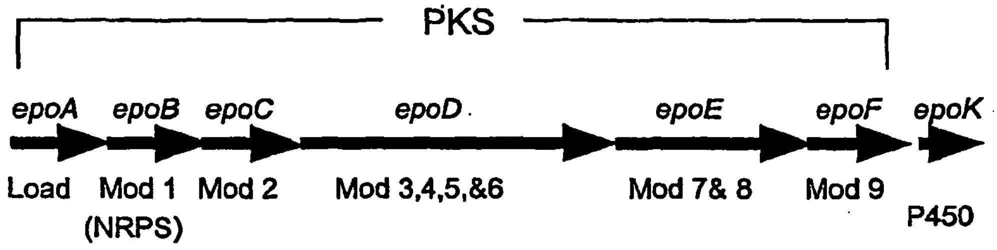


Immediately downstream of *epoK*, the P450 epoxidase gene, is ORF1, which encodes a polypeptide that appears to include membrane spanning domains and may be involved in epothilone transport. This ORF is followed by a number of ORFs that include genes that may encode proteins involved in transport and regulation.

A detailed examination of the modules shows an organization and composition consistent with the biosynthesis of epothilone. The description that follows is at the polypetide leveL The sequence of the AT domain in the loading module and in extender modules 3, 4, 5, and 9 shows similarity to the consensus sequence for malonyl loading modules,

consistent with the presence of an H side chain at C-14, C-12 (epothilones A and C), C-10, and C-2, respectively, as well as the loading module. The AT domains in modules 2, 6, 7, and 8 resemble the consensus sequence for methylmalonyl specifying AT domains, again consistent with the presence of methyl side chains at C-16, C-8, C-6, and C-4 respectively.

The loading module contains a KS domain in which the cysteine residue usually present at the active site is instead a tyrosine. This domain is designated as KSy and serves as a decarboxylase, which is part of its normal function, but cannot function as a condensing enzyme. Thus, the loading module is expected to load malonyl CoA, move it to the ACP, and decarboxylate it to yield the acetyl residue required for condensation with cysteine. Extender module 1 is the non-ribosomal peptide synthetase that activates cysteine and catalyzes the condensation with acetate on the loading module. The sequence contains segments highly similar to ATP-binding and ATPase domains, required for activation of amino acids, a phosphopantetheinylation site, an oxidation domain, a cyclization domain, and an elongation domain. Extender module 2 determines the structure of epothilone at C-15 - C-17. The presence of the DH domain in module 2 yields the C-16-17 dehydro moiety in the molecule. The domains in module 3 are consistent with the structure of epothilone at C-14 and C-15; the OH that comes from the action of the KR is employed in the lactonization of the molecule. Extender module 4 controls the structure at C-12 and C-13 where a double bond is found in epothilones C and D. Although the sequence of the AT domain appears to resemble those that specify malonate loading, it can also load methylmalonate, thereby accounting in part for the mixture of epothilones found in the fermentation broths of the naturally producing organisms.

A significant departure from the expected array of functions was found in extender module 4. This module was expected to contain a DH domain, thereby directing the synthesis of epothilones C and D as the products of the PKS. Analysis revealed that the space between the AT and KR domains of module 4 was not large enough to accommodate a functional DH domain. Thus, the extent of reduction at module 4 appears not to proceed beyond the ketoreduction of the beta-keto formed after the condensation directed by extender module 4. As shown herein, the epothilone PKS genes alone are sufficient to confer the ability to produce epothilones C and D to the host cells. The heterologous production of epothilones C and D demonstrates that there must be a dehydratase function that introduces the double bond. Based on heterologous expression of the epothilone PKS genes and the products produced by altered epothilone PKS genes, the dehydration reaction that forms this double bond is believed to be mediated by the DH domain of extender module 5 of the epothilone PKS and the generation of a conjugated diene precursor prior to reduction by the ER domain of module 5.

Extender modules 5 and 6 each have the full set of reduction domains (KR, DH and ER) to yield the methylene functions at C-11 and C-9. Extender modules 7 and 9 have KR domains to yield the hydroxyls at C-7 and C-3, and extender module 8 does not have a functional KR domain, consistent with the presence of the keto group at C-5. Extender module 8 also contains a methyltransferase (MT) domain that results in the presence of the geminal dimethyl function at C-4. Extender module 9 also has a thioesterase domain that terminates polyketide synthesis and catalyzes ring closure.

The genes, proteins, modules, and domains of the epothilone PKS are summarized in the following Table 2.

**TABLE 2**

| Gene | Protein | Modules | Domains Present |
|---|---|---|---|
| *epoA* | EpoA | Load | $KS^Y$ mAT ER ACP |
| *epoB* | EpoB | 1 | NRPS, condensation, heterocyclization, |
| | | | adenylation, thiolation, PCP |
| *epoC* | EpoC | 2 | KS mmAT DH KR ACP |
| *epoD* | EpoD | 3-6 | KS mAT KR ACP; KS mAT KR ACP; KS |
| | | | mAT DH ER KR ACP; KS mmAT DH ER |
| | | | KR ACP |
| *epoE* | EpoE | 7-8 | KS mmAT KR ACP; KS mmAT MT DH* KR* ACP |
| *epoF* | EpoF | 9 | KS mAT KR DH* ER* ACP TE |
| NRPS - non-ribosomal peptide synthetase; KS - ketosynthase; mAT - malonyl CoA specifying acyltransferase; mmAT - methylmalonyl CoA specifying acyltransferase; DH - dehydratase; ER - enoylreductase; KR - ketoreductase; MT - methyltransferase; TE thioesterase; * - inactive domain. | | | |

Inspection of the sequence has revealed translational coupling between *epoA* and *epoB* (loading module and

the extender module 1 NRPS) and between *epoC* and *epoD*. Very small gaps are seen between *epoD* and *epoE* and *epoE* and *epoF* but gaps exceeding 100 bp are found between *epoB* and *epoC* and *epoF* and *epoK*. These intergenic regions may contain promoters.

Thus, the epothilone PKS is a multiprotein complex composed of the gene products of the *epoA, epoB, epoC, epoD, epoE*, and *epoF* genes. To confer the ability to produce epothilones to a host cell, one provides the host cell with the recombinant *epoA, epoB, epoC, epoD, epoE*, and *epoF* genes, and optionally other genes, such as *epoK*, capable of expression in that host cell. Those of skill in the art will appreciate that, while the epothilone and other PKS enzymes may be referred to as a single entity herein, these enzymes are typically multisubunit proteins. Thus, one can make a derivative PKS (a PKS that differs from a naturally occurring PKS by deletion or mutation) or hybrid PKS (a PKS that is composed of portions of two different PKS enzymes) by altering one or more genes that encode one or more of the multiple proteins that constitute the PKS.

The post-PKS modification or tailoring of epothilone includes multiple steps mediated by multiple enzymes. These enzymes are referred to herein as tailoring or modification enzymes. Expression of the epothilone PKS genes *epoA, epoB, epoC, epoD, epoE*, and *epoF* in host cells that do not express *epoK* leads to the production of epothilones C and D as major products, which lack the C-12-C-13 epoxide of epothilones A and B, having instead a C-12-C-13 double bond. Thus, epothilones C and D are converted to epothilones A and B by an epoxidase encoded by the *epoK* gene. Epothilones A and B may be converted to epothilones E and F by a hydroxylase gene, which may be encoded by a gene associated with the epothilone PKS gene cluster or by another gene endogenous to *Sorangium cellulosum.* Alternatively, these compounds may be formed by the loading module binding a starter unit other than malonyl CoA (such as hydroxymalonyl CoA). Thus, one can produce an epothilone or epothilone derivative modified as desired in a host cell by providing that host cell with one or more recombinant modification enzyme genes provided by the invention or by utilizing a host cell that naturally expresses (or does not express) the modification enzyme and/or by providing starter units other than malonyl CoA.

A wide variety of recombinant DNA compounds and host cells may be used for expressing the naturally occurring epothilones A, B, C, and D and derivatives thereof. Recombinant host cells may produce epothilone derivatives modified in a manner similar to epothilones E and F. Moreover, any epothilone or epothilone derivative can be converted to the corresponding epothilone E or F derivative in accordance with the methods described in PCT Pat Pub. No. 00/039276,. .

The host cells described herein can be grown and fermented under conditions known in the art for other purposes to produce the compounds. The compounds can be isolated from the fermentation broths of these cultured cells and purified by methods such as those in Example 3, below.

Methods for purifying epothilones from fermentation media and for preparing crystalline forms of epothilone are described herein. In general, the purification method involves capture of the epothilone onto XAD resin during fermentation, elution from the resin, solid phase extraction, chromatography, and crystallization. The method is described in detail in Example 3, and while the method is preferred and exemplified for epothilone D, the method can be used to prepare crystalline epothilones generally, including but not limited to other naturally occurring epothilones, and epothilone analogs produced by the host cells described herein.

Epothilone derivative compounds may be provided in isolated and purified forms useful in agriculture, veterinary practice, and medicine. The compounds may be useful as fungicides. The compounds may be useful in cancer chemotherapy. The compounds may be useful for the prevention of undesired cell growth, including but not limited to the treatment of hyperproliferative diseases such as inflammation, autoimmune disease, and psoriasis, and to the prevention of cell growth in stents. The compounds may be useful as immunosuppressants. The compounds may be useful in the manufacture of another compound. The compounds may be formulated in a mixture or solution for administration to a human or animal.

Epothilones produced by the host cells described herein, can be derivatized and formulated as described in PCT patent publication Nos. 93/10121, 97/19086, 98/08849, 98/22461, 98/25929, 99/01124, 99/02514, 99/07692, 99/27890, 99/39694, 99/40047, 99/42602, 99/43320, 99/43653, 99/54318, 99/54319, 99/54330, 99/65913, 99/67252, 99/67253, and 00/00485, and U.S. Patent No. 5,969,145.

## Example 1

## Construction of a *Myxococcus xanthus* Expression Vector

The DNA providing the integration and attachment function of phage Mx8 was inserted into commercially available pACYC184 (New England Biolabs). An ~2360 bp *Mfe*I-*Sma*I from plasmid pPLH343, described in Salmi et al., Feb. 1998, J. Bact. 180(3): 614-621, was isolated and ligated to the large *Eco*RI-*Xmn*I restriction fragment of plasmid pACYC184. The circular DNA thus formed was ~6 kb in size and called plasmid pKOS35-77.

Plasmid pKOS35-77 serves as a convenient plasmid for expressing recombinant PKS genes under the control of the epothilone PKS gene promoter. In one illustrative embodiment, the entire epothilone PKS gene with its homologous

promoter is inserted in one or more fragments into the plasmid to yield an expression vector.

Expression vectors may be provided in which the recombinant PKS genes of the invention are under the control of a *Myxococcus xanthus* promoter. To construct an illustrative vector, the promoter of the *pilA* gene of *M. xanthus* was isolated as a PCR amplification product. Plasmid pSWU357, which comprises the *pilA* gene promoter and is described in Wu and Kaiser, Dec. 1997, *J. Bact. 179*(24):7748-7758, was mixed with PCR primers Seq1 and Mxpil1 primers:

Seq1: 5'-AGCGGATAACAATTTCACACAGGAAACAGC-3'; and
Mxpil1: 5'-TTAATTAAGAGAAGGTTGCAACGGGGGGC-3', and amplified using standard PCR conditions to yield an ~800 bp fragment. This fragment was cleaved with restriction enzyme *KpnI* and ligated to the large *KpnI-EcoRV* restriction fragment of commercially available plasmid pLitmus 28 (New England Biolabs). The resulting circular DNA was designated plasmid pKOS35-71B.

The promoter of the *pilA* gene from plasmid pKOS35-71B was isolated as an ~800 bp *Eco*RV-*Sna*BI restriction fragment and ligated with the large *Msc*I restriction fragment of plasmid pKOS35-77 to yield a circular DNA ~6.8 kb in size. Because the ~800 bp fragment could be inserted in either one of two orientations, the ligation produced two plasmids of the same size, which were designated as plasmids pKOS35-82.1 and pKOS35-82.2. Restriction site and function maps of these plasmids are presented in Figure 1.

Plasmids pKOS35-82.1 and pKOS35-822 serve as convenient starting materials for the vectors in which a recombinant PKS gene is placed under the control of the *Myxococcus xanthus pilA* gene promoter. These plasmids comprise a single *PacI* restriction enzyme recognition sequence placed immediately downstream of the transcription start site of the promoter. In one illustrative embodiment, the entire epothilone PKS gene without its homologous promoter is inserted in one or more fragments into the plasmids at the *PacI* site to yield expression vectors.

The sequence of the *pilA* promoter in these plasmids is shown below.

CGACGCAGGTGAAGCTGCTTCGTGTGCTCCAGGAGCGGAAGGTGAAGCCG
GTCGGCAGCGCCGCGGAGATTCCCTTCCAGGCGCGTGTCATCGCGGCAAC
GAACCGGCGGCTCGAAGCCGAAGTAAAGGCCGGACGCTTTCGTGAGGACC
TCTTCTACCGGCTCAACGTCATCACGTTGGAGCTGCCTCCACTGCGCGAGC
GTTCCGGCGACGTGTCGTTGCTGGCGAACTACTTCCTGTCCAGACTGTCGG
AGGAGTTGGGGCGACCCGGTCTGCGTTTCTCCCCCGAGACACTGGGGCTAT
TGGAGCGCTATCCCTTCCCAGGCAACGTGCGGCAGCTGCAGAACATGGTG
GAGCGGGCCGCGACCCTGTCGGATTCAGACCTCCTGGGGCCCTCCACGCTT
CCACCCGCAGTGCGGGGCGATACAGACCCCGCCGTGCGTCCCGTGGAGGG
CAGTGAGCCAGGGCTGGTGGCGGGCTTCAACCTGGAGCGGCATCTCGACG
ACAGCGAGCGGCGCTATCTCGTCGCGGCGATGAAGCAGGCCGGGGGCGTG
AAGACCCGTGCTGCGGAGTTGCTGGGCCTTTCGTTCCGTTCATTCCGCTACC
GGTTGGCCAAGCATGGGCTGACGGATGACTTGGAGCCCGGGAGCGCTTCG
GATGCGTAGGCTGATCGACAGTTATCGTCAGCGTCACTGCCGAATTTTGTC
AGCCCTGGACCCATCCTCGCCGAGGGGATTGTTCCAAGCCTTGAGAATTGG
GGGGCTTGGAGTGCGCACCTGGGTTGGCATGCGTAGTGCTAATCCCATCCG
CGGGCGCAGTGCCCCCCGTTGCAACCTTCTCTTAATTAA

To make recombinant *Myxococcus xanthus* host cells, *M. xanthus* cells are grown in CYE media (Campos and Zusman, 1975, Regulation of development in Myxococcus xanthus: effect of 3': 5'-cyclic AMP, ADP, and nutrition, Proc.

Natl. Acad. Sci. USA 72: 518-522) to a Klett of 100 at 30°C at 300 rpm. The remainder of the protocol is conducted at 25°C unless otherwise indicated. The cells are then pelleted by centrifugation (8000 rpm for 10 min. in an SS34 or SA600 rotor) and resuspended in deionized water. The cells are again pelleted and resuspended in 1/100th of the original volume.

DNA (one to two μL) is electroporated into the cells in a 0.1 cm cuvette at room temperature at 400 ohm, 25 μFD, 0.65 V with a time constant in the range of 8.8 - 9.4. The DNA is free of salts and is resuspended in distilled and deionized water or dialyzed on a 0.025 μm Type VS membrane (Millipore). For low efficiency electroporations, the DNA is spot dialyzed, and outgrowth is in CYE. Immediately after electroporation, 1 mL of CYE is added, and the cells in the cuvette pooled with an additional 1.5 mL of CYE previously added to a 50 mL Erlenmeyer flask (total volume 25 ml). The cells are grown for four to eight hours (or overnight) at 30 to 32°C at 300 rpm to allow for expression of the selectable marker. Then, the cells are plated in CYE soft agar on plates with selection. With kanamycin as the selectable marker, typical yields are $10^3$ to $10^5$ per μg of DNA. With streptomycin as the selectable marker, it is included in the top agar, because it binds agar.

With this procedure, the recombinant DNA expression vectors are electroporated into *Myxococcus* host cells that express recombinant PKSs and produce the epothilone, epothilone derivatives, and other polyketides encoded thereby.

Example 2

Chromosomal Integration and a Bacterial Artificial Chromosome (BAC) for Expression of Epothilone in *Myxococcus xanthus*

To express the epothilone PKS and modification enzyme genes in a heterologous host to produce epothilones by fermentation, *Myxococcus xanthus*, which is closely related to *Sorangium cellulosum* and for which a number of cloning vectors are available, is employed. *M. xanthus* and *S. cellulosum* are myxobacteria and so may share common elements of gene expression, translational control, and post translational modification. *M. xanthus* has been developed for gene cloning and expression: DNA can be introduced by electroporation, and a number of vectors and genetic markers are available for the introduction of foreign DNA, including those that permit its stable insertion into the chromosome. *M. xanthus* can be grown with relative ease in complex media in fermentors and can be subjected to manipulations to increase gene expression, if required.

To introduce the epothilone gene cluster into *Myxococcus xanthus*, one can build the epothilone cluster into the chromosome by using homologous recombination to assemble the complete gene cluster. Alternatively, the complete epothilone gene cluster can be cloned on a bacterial artificial chromosome (BAC) and then moved into *M. xanthus* for integration into the chromosome.

To assemble the gene cluster from cosmids pKOS35-70.1A2, and pKOS35-79.85, small regions (~2 kb or larger) of homology from these cosmids are introduced into *Myxococcus xanthus* to provide recombination sites for larger pieces of the gene cluster. As shown in Figure 2, plasmids pKOS35-154 and pKOS90-22 are created to introduce these recombination sites. The strategy for assembling the epothilone gene cluster in the *M. xanthus* chromosome is shown in Figure 3. Initially, a neutral site in the bacterial chromosome is chosen that does not disrupt any genes or transcriptional units. One such region is downstream of the *devS* gene, which has been shown not to affect the growth or development of *M. xanthus*. The first plasmid, pKOS35-154, is linearized with *Dra*I and electroporated into *M. xanthus*. This plasmid contains two regions of the *dev* locus flanking two fragments of the epothilone gene cluster. Inserted in between the epo gene regions is a cassette composed of a kanamycin resistance marker and the *E. coli galK* gene. See Ueki et al., 1996, Gene 183: 153-157,. Kanamycin resistance arises in colonies if the DNA recombines into the *dev* region by a double recombination using the *dev* sequence as regions of homology.

This strain, K35-159, contains small (~2.5 kb) regions of the epothilone gene cluster that will allow for recombination of pKOS35-79.85. Because the resistance markers on pKOS35-79.85 are the same as that in K35-159, a tetracycline transposon was transposed into the cosmid, and cosmids that contain the transposon inserted into the kanamycin marker were selected. This cosmid, pKOS90-23, was electroporated into K35-159, and oxytetracycline resistant colonies were selected to create strain K35-174. To remove the unwanted regions from the cosmid and leave only the epothilone genes, cells were plated on CYE plates containing 1% galactose. The presence of the *galK* gene makes the cells sensitive to 1% galactose. Galactose resistant colonies of K35-174 represent cells that have lost the *galK* marker by recombination or by a mutation in the *galK* gene. If the recombination event occurs, then the galactose resistant strain is sensitive to kanamycin and oxytetracycline. Strains sensitive to both antibiotics are verified by Southern blot analysis. The correct strain is identified and designated K35-175 and contains the epothilone gene cluster from module 7 to 4680 bp downstream of the stop codon of *epoK*.

To introduce modules 1 through module 7, the above process is repeated once more. The plasmid pKOS90-22 is linearized with *Dra*I and electroporated into K35-175 to create K111-13.2. This strain is electroporated with the tetracycline resistant version of pKOS35-70.1A2, pKOS90-38, and colonies resistant to oxytetracycline are selected. This

creates strain K111-13.23. Recombinants that now have the whole epothilone gene cluster are selected by resistance to 1% galactose. This results in clones K111-32.25, K111-32.26, and K111-32.35. Strain K111-32.25 was deposited April 14, 2000, with the American Type Culture Collection, Manassas, VA 20110-2209, USA, in compliance with the Budapest Treaty and is available under accession No. PTA-1700. This strain contains all the epothilone genes and their promoter(s).

Fermentation was performed by inoculating strains into 5 mL of CYE (10 g casitone, 5 g yeast extract, and 1 g $MgSO_4$•$7H_2O$ per liter) in a 50 mL flask and growing overnight until the culture was in mid log growth phase. A 100 µL aliquot was spread onto a CTS plate, and the plate incubated at 32°C for 4 to 5 days. To extract epothilones, the agar and cells from the plate was macerated, put in a 50 mL conical tube, and acetone added to fill the tube. The solution was incubated with rocking for 4 to 5 hours, the acetone evaporated, and the remaining liquid extracted twice with an equal volume of ethyl acetate. The water was removed from the ethyl acetate extract by adding magnesium sulfate. The magnesium sulfate was filtered out, and the liquid was evaporated to dryness. The epothilones were resuspended in 200 µL of acetonitrile and analyzed by LC/MS. The analysis showed that the strain produced epothilones A and B, with epothilone B present at about 0.1 mg/L in the culture, and epothilone A at 5 to 10-fold lower levels.

This strain can also be used to produce epothilones in liquid culture. A flask containing CYE is inoculated with an epothilone producing strain. The next day, while the cells are in mid-log growth phase, a 5% inoculum is added to a flask containing 0.5% CMM (0.5% casitone, 0.2% MgSO4•$7H_2O$,10 mM MOPS pH7.6) along with 1 mg/mL serine, alanine, and glycine and 0.1% sodium puyruvate. The sodium pyruvate can be added to 0.5% to increase epothilone B production but causes a decrease in the ratio of epothilone B to epothilone A. The culture is grown at 30°C for 60-72 hours. Longer incubations result in a decrease in titers of epothilones. To recover epothilones, the cultures are centrifuged at 10,000 rpm for 10 minutes in an SS34 rotor. The supernatants are extracted twice with ethyl acetate and rotary evaporated ("rotavated") to dryness. Liquid cultures produced 2 to 3 mg/L of epothilones A and B, with ratios similar to that observed with plate cultures. If XAD (0.5 - 2%) was added to the culture, epothilones C and D were observed, with epothilone D present at 0.1 mg/L and epothilone C present at 5 to 10-fold lower levels.

To clone the whole gene cluster as one fragment, a bacterial artifical chromosome (BAC) library is constructed. First, SMP44 cells are embedded in agarose and lysed according to the BIO-RAD genomic DNA plug kit. DNA plugs are partially digested with restriction enzyme, such as Sau3AI or *Hin*dIII, and electrophoresed on a FIGE or CHEF gel. DNA fragments are isolated by electroeluting the DNA from the agarose or using gelase to degrade the agarose. The method of choice to isolate the fragments is electroelution, as described in Strong et al., 1997, Nucleic Acids Res. 19: 3959-3961,. The DNA is ligated into the BAC (pBeloBACII) cleaved with the appropriate enzyme. A map of pBeloBACII is shown in Figure 4.

The DNA is electroporated into DH10B cells by the method of Sheng et al., 1995, Nucleic Acids Res. 23: 1990-1996, to create a *Sorangium cellulosum* genomic library. Colonies are screened using a probe from the NRPS region of the epothilone cluster. Positive clones are picked and DNA is isolated for restriction analysis to confirm the presence of the complete gene cluster. This positive clone is designated pKOS35-178.

To create a strain that can be used to introduce pKOS35-178, a plasmid, pKOS35-164, is constructed that contains regions of homology that are upstream and downstream of the epothilone gene cluster flanked by the *dev* locus and containing the kanamycin resistance *galK* cassette, analogous to plasmids pKOS90-22 and pKOS35-154. This plasmid is linearized with *Dra*I and electroporated into *Myxococcus xanthus*, in accordance with the method of Kafeshi et al., 1995, Mol. Microbiol. 15: 483-494, to create K35-183. The plasmid pKOS35-178 can be introduced into K35-183 by electroporation or by transduction with bacteriophage P1, and chloramphenicol resistant colonies are selected. Alternatively, a version of pKOS35-178 that contains the origin of conjugative transfer from pRP4 can be constructed for transfer of DNA from *E. coli* to K35-183. This plasmid is made by first constructing a transposon containing the oriT region from RP4 and the tetracycline resistance maker from pACYC184 and then transposing the transposon *in vitro* or *in vivo* onto pKOS35-178. This plasmid is transformed into S171 and conjugated into *M. xanthus*. This strain, K35-190, is grown in the presence of 1% galactose to select for the second recombination event This strain contains all the epothilone genes as well as all potential promoters. This strain is fermented and tested for the production of epothilones A and B.

Alternatively, the transposon can be recombined into the BAC using either the temperature sensitive plasmid peak705 or pKO3 by transposing the transposon onto either pMAK705 or pKO3 selecting for tetR and camS plasmids; the recombination is accomplished as described in Hamilton et al., Sep. 1989, J. Bact.171(9): 4617-4622 and Link et al., Oct 1997, J. Bact.179(20): 6228-6237,.

Besides integrating pKOS35-178 into the *dev* locus, it can also be integrated into a phage attachment site using integration functions from myxophages Mx8 or Mx9 A transposon is constructed that contains the integration genes and *att* site from either Mx8 or Mx9 along with the tetracycline gene from pACYC184. Alternative versions of this transposon may have.only the attachment site. In this version, the integration genes are then supplied in trans by coelectroporation of a plasmid containing the integrase gene or having the integrase protein expressed in the electroporated strain from any constitutive promoter, such as the mgl promoter (see Magrini et al., Jul. 1999, J. Bact. 181(13):

4062-4070,). Once the transposon is constructed, it is transposed onto pKOS35-178 to create pKOS35-191. This plasmid is introduced into *Myxococcus xanthus* as described above. This strain contains all the epothilone genes as well as all potential promoters. This strain is fermented and tested for the production of epothilones A and B. Alternatively, a strain that contains the *att* site and the *oriT* region can be transposed onto the BAC and the resulting BAC conjugated into *M. xanthus*.

Once the epothilone genes have been established in a strain of *Myxococcus xanthus*, manipulation of any part of the gene cluster, such as changing promoters or swapping modules, can be performed using the kanamycin resistance and *galk* cassette, as described below. Cultures *of Myxococcus xanthus* containing the *epo* genes are grown in a number of media and examined for production of epothilones. If the levels of production of epothilones (in particular B or D) are low, then the *M. xanthus*-producing clones are subjected to media development and mutation based strain improvement, as described in the following example.

Example 3

Processes for the Production and Purification of Epothilones

A. Optimizing the Heterologous Production of Epothilone D in Myxococcus xanthus

The heterologous production of epothilone D in *Myxococcus xanthus* was improved by 140-fold from an initial titer of 0.16 mg/L with the incorporation of an adsorber resin, the identification of a suitable carbon source, and the implementation of a fed-batch process.

To reduce the degradation of epothilone D in the basal medium, XAD-16 (20 g/L) was added to stabilize the extracellular product This greatly facilitated its recovery and enhanced the yield by three-fold. The use of oils as a carbon source for cell growth and product formation was also evaluated. From a screen of various oils, methyl oleate was shown to have the greatest impact At the optimal concentration of 7 mL/L in a batch process, the maximum cell density was increased from 0.4 g dry cell weight (DCW)/L to 2 g DCW/L. Product yield depended on the presence of trace elements in the production medium. With an exogenous supplement of trace metals to the basal medium, the peak epothilone D titer was enhanced eight-fold, demonstrating the significant role of metal ions in cell metabolism and in epothilone biosynthesis. To increase the product yield further, a continuous fed-batch process was employed to promote a higher cell density and to maintain an extended production period. The optimized fed-batch cultures consistently yielded a cell density of 7 g DCW/L and an average production titer of 23 mg/L.

Epothilones are secondary metabolites that are naturally produced by various strains of the myxobacterium *Sorangium cellulosum* (Gerth *et al*., 1996; Gerth *et al*., 2001; references cited in this example are listed at the end of this section). They are potent inhibitors of microtubule depolymerization, with a mechanism of action similar to that of the anti-cancer drug Taxol (Bollag *et al*., 1995). Their cytotoxic effect against multiple-drug resistant tumor cell lines expressing the P-glycoprotein renders them potential therapeutic compounds with great commercial value (Su *et al*., 1997; Kowalski *et al*., 1997). Their comparatively high solubility in water also facilitates their formulation for clinical evaluation.

Epothilones A and B are the major fermentation products of the natural host (Gerth *et al*., 1996). The macrocyclic core of these polyketide molecules is formed by the successive decarboxylative condensations of acetate and propionate units (Gerth *et al*., 2000). Epothilones A and B differ by a single methyl group at the C-12 position of their carbon skeleton. This structural variance results from the incorporation of an acetate in the assembly of epothilone A and a propionate in that of epothilone B. Epothilones C and D are intermediates in the biosynthetic pathway of epothilones A and B, respectively (Tang *et al*., 2000; Molnár *et al.;* 2000). They are excreted as minor products during the fermentation process, with a combined yield of about 0.4 mg/L. Because preliminary *in vivo* studies revealed epothilone D to be the most promising of the four compounds as an anti-tumor drug (Chou *et al*., 1998), it is of considerable interest to produce this molecule on a large scale.

The gene cluster responsible for the biosynthesis of the epothilones has been sequenced (Tang *et al.,* 2000; Molnár *et al.,* 2000) and used to produce these compounds in *Myxococcus xanthus*, a microbial host closely-related to *S. cellulosum* but more amenable to genetic manipulation. To foster the production of epothilone D, a deletion mutant of this recombinant strain (described in Example 4, below) was constructed to inactivate the P450 epoxidase that catalyzes the conversion of epothilones C and D to epothilones A and B, respectively (Tang *et al.,* 2000). This genetic alteration effectively promoted the secretion of epothilones C and D as sole products of the *M. xanthus* fermentation, with an epothilone D to C ratio of 4 to 1. The resulting mutant offers a distinct advantage over the natural host in the recovery and purification of the desired product In this example, improvements in media composition and fermentation strategy are described that result in a 140-fold increase in the production of epothilone D in *M. xanthus.*

Adsorber resins have been used in the fermentations of myxobacteria for the continuous capture of biologically active molecules produced at low quantities (Reichenbach and Höfle, 1993). To facilitate the isolation of epothilone D,

the hydrophobic resin XAD-16 was added to the culture medium. Because the bound product can readily be eluted from the resin with an appropriate solvent, its recovery was greatly simplified. Moreover, the use of XAD-16 minimized epothilone degradation through product stabilization.

*Myxococcus xanthus* has been traditionally cultivated in media consisting primarily of enzymatic hydrolysates of casein, such as peptone and casitone, relying on amino acids as the sole carbon and nitrogen source (Reichenbach and Dworkin 1991). Consequently, ammonia is accumulated in the fermentation broth as a result of amino acid degradation. It was demonstrated by Gerth *et al.* (1986) that an extracellular ammonia concentration of 35-42 mM in a *Myxococcus virescens* culture corresponded to a surprisingly high ammonia concentration of 80-140 mM within the cells. More importantly, it was shown that by continuously removing the excess ammonia to below 8 mM with an *in situ* membrane process, both cell mass and secondary metabolite production dramatically increased (Hecht *et al.*, 1990). Because the generation of high levels of ammonia is speculated to be inhibitory to the growth of *M. xanthus* and epothilone D production, an alternative carbon source to reduce the consumption of amino acids is desirable.

Although an adaptation process was required, methyl oleate was identified from an extensive screen of different oils as a substrate that can be metabolized by *M. xanthus*. With the addition of an exogenous trace element solution to the growth medium, epothilone D production was enhanced 8-fold, with a yield of 3.3 mg/L in a simple batch fermentation. To optimize the process further, a fed-batch approach using intermittent or continuous feeds of casitone and methyl oleate was adopted to prolong the production phase of the cells. A comparison of the results obtained with the two different feed strategies is reported in this example.

## MATERIALS AND METHODS

### *Inoculum Preparation*

For the production of epothilone D in culture media without methyl oleate. 1 mL of frozen cells of the *Myxococcus xanthus* strain K111-40.1 in 20 % (v/v) glycerol was inoculated into 3 mL of CYE medium consisting of 10 g/L casitone (Difco), 5 g/L yeast extract (Difco),1 g/L $MgSO_4$-$7H_2O$, and 50 mM HEPES, pH 7.6, in a 50-mL glass culture tube. The HEPES buffer solution was titrated to pH 7.6 with potassium hydroxide. The cells were incubated at 30°C and 175 rpm on a rotary shaker for 3 days. They were then transferred to a 250-mL Erlenmeyer flask containing 50 mL of CYE medium and grown for 2 days under the same conditions. The resulting seed culture was used to inoculate 50-mL production flasks at an inoculum size of 5% (v/v).

For the cultivation of *M. xanthus* in media containing methyl oleate, the cells had to be adapted to growth in the presence of the oil. One seed vial of frozen cells was inoculated into 3 mL of CYE medium that was supplemented with 3 μL of methyl oleate (Emerest 2301) (Cognis Corp.). The cells were grown in a glass culture tube for 2-6 days at 30°C and 175 rpm until the culture was sufficiently dense under a microscope. They were then transferred into a 250 mL Erlenmeyer flask containing 50 mL of CYE-MOM medium consisting of 10 g/L casitone (Difco), 5 g/L yeast extract (Difco),1 g/L $MgSO_4 \cdot 7H_2O$, 2 mL/L methyl oleate, and 50 mM HEPES, pH 7.6. After 2 days of growth, the cells were frozen and stored at -80°C as 1 mL aliquots in 20% (v/v) glycerol.

For the production of epothilone D in media containing methyl oleate, 1 mL of the frozen oil-adapted cells was inoculated into 3 mL of CYE-MOM medium in a glass culture tube. The cells were incubated at 30°C and 175 rpm. for 2 days and transferred to a 250 mL Erlenmeyer flask containing 50 mL of CYE-MOM medium. The resulting seed culture was grown for 2 days under the same conditions and was used to inoculate 50 mL production flasks at an inoculum size of 5% (v/v).

In preparing the inoculum for 5-L fermentations, 25 mL of the oil-adapted seed culture were transferred into a 28 L Fernbach flask containing 475 mL of CYE-MOM medium. The cells were grown at 30°C and 175 rpm for 2 days. Subsequently, 250 mL of this secondary seed culture was inoculated into 5-L fermentors containing 4.75 L of production medium to yield a final inoculum concentration of 5% (v/v).

### *Shake Flask Production*

Batch cultivations of *M. xanthus* K111-40-1 in the absence of methyl oleate were prepared as follows. One gram of XAD-16 resin (Rohm and Haas) was autoclaved at 121°C for 30 min in a 250-mL Erlenmeyer flask with 5 mL of deionized water. The excess water was then removed from the flask, and 50 mL of CTS medium consisting of 5 g/L casitone, 2 g/L $MgSO_4 \cdot 7H_2O$, and 50 mM HEPES, pH 7.6, were added. Because autoclaving of the adsorber resin in the presence of the production medium led to the binding of essential nutrients required by the cells, the resin and medium components were sterilized separately. The production flasks were inoculated with 2.5 mL of seed culture and incubated at 30°C and 175 rpm for 6 days.

Batch cultivations in the presence of methyl oleate were prepared as described above. In addition, the production medium was supplemented with 7 mL/L of methyl oleate and 4 mL/L of a filter-sterilized trace element solution that was

composed of 10 mL/L concentrated HaSO4,14.6 g/L FeCl3-6$H_2O$,2.0 g/L $ZnCl_3$, 1.0 g/L $MnCl_2$-4$H_2O$, 0.43 g/L $CuCl_2 \cdot 2H_2O$, 0.31 g/L $H_3BO_3$, 0.24 g/L $CaCl_2$-6$H_2O$, and 0.24 g/L $Na_2$MO4.2$H_2O$. The production flasks were then inoculated with 25 mL of the oil-adapted seed culture and grown at 30°C and 175. rpm for 5 days.

Fed-batch cultures with intermittent feeds of casitone and methyl oleate were prepared as follows. One gram of XAD-16 resin was autoclaved at 121°C for 30 min. in a 250 mL Erlenmeyer flask with 5 mL of deionized water. After sterilization, the excess water was removed from the flask, and 50 mL of CTS medium supplemented with 2 mL/L methyl oleate, 4 mL/L trace element solution, and 50 mM HEPES, pH 7.6, were added. The production flasks were inoculated with 2.5 mL of the oil-adapted seed culture and incubated at 30°C and 175 rpm. Two days after inoculation, 2 g/L of casitone and 3 mL/L of methyl oleate were added to the culture medium at 24 h intervals. The casitone feed was prepared as a concentrated 100 g/L solution. The cultures were grown for 10-12 days until substantial cell lysis was observed.

All the production cultures can be grown on a 500-mL scale in 28-L Fernbach flasks under the same growth conditions.

*Fementor Production*

Fed-batch fermentations on a 5-L scale with intermittent or continuous feeds of casitone and methyl oleate were prepared as follows. Twenty grams per liter of XAD-16 and 2 g/L of $MgSO_4 \cdot 7H_2O$ was autoclaved at 121°C for 30 min in a 5-L fermentor (B. Braun) with 4.75 L of deionized water. After sterilization, a concentrated casitone solution (150 g/L), methyl oleate, and trace elements were added to the bioreactor aseptically to attain a final casitone, methyl oleate, and trace element concentration of 5 g/L, 2 mL/L, and 4 mL/L, respectively. The medium was then inoculated with 250 mL of the oil-adapted seed culture. The fermentation was performed at 30°C with an aeration rate of 0.4-0.5 v/v/m and an initial agitation rate of 400 rpm. The dissolved oxygen was controlled at 50% of saturation by a stirring cascade between 400-700 rpm. Cultivation pH was maintained at 7.4 by the automated addition of 2.5 N KOH and 2.5 $MH_2SO_4$. Twenty-four hours after inoculation, casitone (150 g/L) and methyl oleate were added to the production medium at a feed rate of 2 g/L/day casitone and mL/L/ day methyl oleate. The feeds were delivered either as a single bolus every 24 h or continuously with peristaltic pumps (W. Maxlow). The cells were allowed to grow for 10-12 days until considerable cell lysis was noted.

*Epothilone Quantitation*

Prior to the use of the XAD-16 resin in the fermentations, 1 mL of culture broth was sampled from the production flasks or bioreactors and centrifuged at 13,000 g for 10 min. Quantitation of the epothilone products in the supernatant was carried out using a Hewlett Packard 1090 HPLC with UV detection at 250 nm Five hundred microliters of the supernatant were injected across a 4.6 x 10 mm guard column (Inertsil, ODS-3, 5 μm). An online extraction was then performed at a flow rate of 1 mL/min. with a 100% water wash for 0.5 min., followed by a gradient to 50% acetonitrile over 1.5 min. The eluant was diverted to waste for the first two minutes and was passed onto a longer separation column (4.6 x 150 mm, Inertsil, ODS-3, 5 μm) thereafter. Separation of epothilones C and D was performed with a gradient from 50% to 100% acetonitrile over 8 min, followed by a 100% acetonitrile wash for 3 min. Under these conditions, epothilone C eluted at 9.4 minutes and epothilone D eluted at 9.8 minutes.

With the use of the XAD-16 resin, 5-50 mL of well-mixed culture broth and resin were sampled from the production flasks or bioreactors. After the resin was settled by gravity, the culture broth was decanted. The resin was washed with 5-50 mL of water and allowed to settle by gravity again. The aqueous mixture was completely removed, and the epothilone products were extracted from the resin with 100% methanol. The amount of solvent used was equivalent to 50% of the sample volume. Quantitation of epothilones C and D was carried out by HPLC analysis with UV detection at 250 nm. Fifty microliters of the methanol extract were injected across two 4.6 x 10 mm guard columns (Inertsil, ODS-3, 5 μm) and a longer 4.6 x 150 mm column (Inertsil, ODS-3,5 μm). The assay method was isocratic, eluting with 60% acetonitrile and 40% water for 18 min at a flow rate of 1. mL/min. Under these conditions, epothilone C was detected at 10.3 minutes and epothilone D was detected at 13.0 minutes. Standards were prepared using purified epothilone D.

*Cell Growth Determination*

Cell growth in the absence of methyl oleate was monitored by measuring the optical density (OD) at 600 nm. Samples were diluted with water until the final OD values were less than 0.4. Because the addition of methyl oleate to culture medium results in the formation of an emulsion that has a strong absorbance at 600 nm, cell growth in the presence of methyl oleate was determined by dry cell weight (DCW). Forty milliliters of culture broth were centrifuged at 4200 g for 20 min in preweighed test tubes. The pellets were then washed with 40 mL of water and dried for 2 days at 80°C before weighing.

*Ammonia Determination*

One milliliter of fermentation broth was clarified by centrifugation at 13,000 g for 5 min. The supernatant was then used for ammonia analysis with an ammonia assay kit (Sigma). Samples were diluted 20-100 fold with water until the final concentrations were less than 880 μM.

*Methyl Oleate Determination*

The residual methyl oleate in 1-mL of fermentation broth was extracted with 5 mL of acetonitrile. The mixture was vortexed and clarified by centrifugation at 4200 g for 20 min. Quantitation of methyl oleate was carried out by HPLC analysis with UV detection at 210 nm. Fifty microliters of the supernatant were injected across two 4.6 x 10 mm guard columns (Inertsil, ODS-3, 5 μm) and a longer 4.6 x 150 mm column (Inertsil, ODS-3, 5 μm). The column was washed with acetonitrile-water (1:1) for 2 min. at a flow rate of 1 mL/min. It was then eluted with a gradient of 50% to 100% acetonitrile over 24 min., followed by a 100% acetonitrile wash for 5 min. Because of the heterogeneity of the carbon chain lengths of commercial methyl oleate, this compound was eluted as two main peaks that were detected at 25.3 minutes and 27.1 minutes. Methyl oleate bound to the XAD-16 resin was quantitated from the methanol extract using the same HPLC method. Standards of methyl oleate were prepared in 83.3% acetonitrile. Consumption of methyl oleate by the cells was calculated as: (total methyl oleate added) - (residual methyl oleate in medium) - (methyl oleate bound to resin).

RESULTS

The *Myxococcus xanthus* strain K111-40-1 was initially cultivated in a batch fermentation process with a simple production medium consisting only of 5 g/L casitone (a pancreatic casein digest) and 2 g/L magnesium sulfate. The baseline performance of the cells is shown in Figure 5.

Maximum cell density and epothilone D production were attained three days after inoculation at an $OD_{600}$ of 1.6 and a corresponding titer of 0.16 mg/L. Both cell density and product yield decreased substantially thereafter. With the consumption of casitone by the cells, a gradual accumulation of ammonium was also detected in the production medium. The final ammonia concentration approached 20 mM at the end of the 5-day fermentation.

*Effect of XAD-16 on product stability*

To prevent the rapid degradation of epothilone D, the hydrophobic adsorber resin, XAD-16, was added to the production medium to bind and stabilize the excreted product XAD-16 is a polyaromatic resin that had previously been used by Gerth *et al.* (1996) for the isolation of epothilones A and B from fermentations of the microbial producer, *Sorangium cellulosum* So ce90. As shown in Figure 6, the presence of the adsorber resin did not affect the growth of the cells. However, it effectively reduced the loss of epothilone D in the fermentation broth, which led to a three-fold enhancement in the recovery of this product

*Media Development*

In an effort to develop a medium that can support higher cell density and epothilone production, the influence of casitone on growth and product yield was evaluated by varying its concentration from 1 g/L to 40 g/L in the production medium. Although cell growth was stimulated with increasing casitone concentrations, the specific productivity of the cells declined significantly, as shown in Figure 7. The optimal casitone concentration for epothilone D production was reached at 5 g/L, with higher concentrations resulting in decreased titers.

Because media improvements were limited with the use of casitone, alternative substrates were evaluated as supplements to the basal production medium. From a detailed screen of different oils, methyl oleate was identified as a carbon source that promoted the greatest increase in epothilone D production, as summarized in Table 3.

**TABLE 3**

| Oil (7 mLL) | Epothilone D production relative to control with no oil supplements (%) |
|---|---|
| Methyl Oleate | 780 |
| Ethyl Oleate | 740 |
| Coconut Oil | 610 |

(continued)

| Oil (7 mLL) | Epothilone D production relative to control with no oil supplements (%) |
|---|---|
| Lard | 470 |
| Propyl Oleate | 420 |
| Sesame Oil | 380 |
| Glycerol Tri-oleate | 370 |
| Salad Oil | 360 |
| Sunflower Oil | 330 |
| Soy Oil | 290 |
| Methyl Heptadecanoate | 190 |
| No Oil (Control) | 100 |
| Methyl Nanadecanoate | 96 |
| Methyl Pelargoante | 40 |
| Rapeseed Oil | 40 |

However, the direct addition of methyl oleate to the production medium resulted in premature cell lysis. Therefore, the seed cultures were grown in the presence of methyl oleate prior to the production fermentations. Interestingly, this adaptation process rendered the cells less susceptible to lysis. As shown in Table 4 (Improvements in Growth and Production Compared to Baseline Performance in CTS Medium in Batch Fermentation), a peak biomass concentration of 21 g/L and an epothilone D titer of 3.3 mg/L were achieved with a methyl oleate concentration of 7 mL/L and a trace elements concentration of 4 mL/L.

**TABLE 4**

| Fermentation Conditions | Maximum Cell Density (g DCW/L) | Maximum Epothilone D Production (mg/L) |
|---|---|---|
| CTS medium with no XAD-16 in batch process | 0.44 ± 0.04 | 0.16 ± 0.03 |
| CTS medium with XAD-16 in batch process | 0.44 ± 0.04 | 0.45 ± 0.09 |
| CTS medium with 7 mL/L methyl oleate in batch process | 1.2 ± 0.1 | 0.12 ± 0.02 |
| CTS medium with 7 mL/L methyl oleate and 4 mL/L trace elements in batch process | 2.1 ± 0.2 | 3.3 ± 0.7 |
| Intermittent fed-batch process | 6.3 ± 0.6 | 9.8 ± 2.0 |
| Continuous fed-batch process | 7.3 ± 0.7 | 23 ± 4.6 |

Further titer improvements were not observed at higher methyl oleate concentrations, as shown in Figure 8.

In addition to demonstrating the significance of methyl oleate on cell growth and production, the above graph also emphasizes the importance of trace elements in the production medium. In anticipation that the nutrients supplied by casitone may not be sufficient for vigorous cell growth, an exogenous addition of trace metals was added in conjunction with the methyl oleate. Surprisingly, this supplement was found to be essential for both growth and production enhancement. In its absence, the maximum biomass concentration and epothilone D titer were only 1.2 g/L and 0.12 mg/L, respectively. This low titer was comparable to that obtained with the basal medium.

*Fed-Batch Development*

In the presence of optimal concentrations of methyl oleate and trace elements in a batch fermentation process, exponential growth of the *M. xanthus* strain occurred during the first two days after inoculation. Production of epothilone D began at the onset of the stationary phase and ceased when cell lysis occurred with the depletion of methyl oleate

on day 5, as shown in Figures 9A and 9B. The time courses for methyl oleate consumption and ammonia generation are also shown. The concentration of ammonia in the production medium was < 4 mM throughout the course of the fermentation.

To extend the production period of the cells in the flask cultures, casitone and methyl oleate were added to the medium once a day at a rate of 2 g/L/day and 3 mL/L/day, respectively. The substrate feeds were initiated 48 h after inoculation, and the cells grew exponentially for three days thereafter. As shown in Figure 10A, the biomass concentration began to plateau on day 5 and reached a maximum at 6.3 g/L on day 10. Again, epothilone D production coincided with the stationary growth phase, and a final yield of 9.8 mg/L was attained at the end of day 12 As shown in the Figure 10B, both the consumption of methyl oleate and the generation of ammonia increased at constant rates over the course of the fermentation.

Methyl oleate was depleted at the same rate it was fed to the bioreactor, and ammonia accumulated at a rate of 3.2 mM/day. Lower feed rates of casitone or methyl oleate greatly reduced the epothilone D titer, while higher feed rates led to the premature lysis of the cells before significant production was achieved.

To test the effectiveness of the fed-batch process on a larger scale, casitone and methyl oleate were added intermittently at 24-h intervals to a 5-L fermentation in a bioreactor. As shown in Figure 11, the resulting production curve closely resembled that for the flask cultures. The substrate feeds were initiated 24 h after inoculation, and the production of epothilone began on day 4. A peak epothilone D titer of 9.2 mg/L was obtained ten days after inoculation.

To assess the impact of a more refined feeding strategy on growth and production, the dual feeds were delivered continuously to the bioreactor. As illustrated in Figure 12A, the implementation of the continuous feeds did not affect the growth of the cells, but it increased their productivity by nearly three-fold. A final epothilone D titer of 27 mg/L was achieved 10 days after inoculation. As shown in Figure 12B, methyl oleate was consumed at the same rate it was added to the production medium, and ammonia was released at a steady rate of 6.4 mM/ day over the course of the fermentation.

DISCUSSION

Although the chemical synthesis of epothilone D has recently been achieved (Harris *et al*., 1999; Meng *et al*., 1998; Sinha *et al*., 1998), the complex 20-step process is not an economically viable method for the large-scale production of the compound. While the initial production yield for epothilone D in *Myxococcus xanthus* strain was 0.16 mg/L, the improved fermentation processes of the invention substantially increased the production level to 23 mg/L.

One of the major barriers to attaining a higher epothilone D titer was rapid degradation of the product in the fermentation broth. This problem was alleviated with the incorporation of an adsorber resin to the culture medium The addition of XAD-16 did not affect the growth of the cells but minimized the loss of the excreted product and increased its recovery by three-fold.

Another obstacle to enhancing the production yield is the limited improvement in titer with the use of casitone as the primary carbon and nitrogen source. Although growth of the *M. xanthus* strain was stimulated with increasing casitone concentrations, a concentration that exceeded 5 g/L resulted in a dramatic decrease in titer. This inhibitory effect in secondary metabolite production at high concentrations of peptone or casitone has previously been demonstrated in several other myxobacterial fermentations and has been attributed to the accumulation of ammonia in the culture medium.

Because *M. xanthus* is incapable of metabolizing polysaccharides and sugars (Reichenbach and Dworkin, 1991), its ability to utilize oils as a carbon source was examined Oils are attractive carbon substrates, because the oxidation of fatty acids not only can serve as a source of energy for the cells, but the formation of acetyl-CoA as a degradation product can also provide precursors for epothilone biosynthesis. The addition of oils to the fermentation of *Saccharopolyspora erythraea, Streptomyces fradiae,* and *Streptomyces hygroscapicus* has been shown to enhance the production of polyketide molecules, such as erythromycin, tylosin, and the immunoregulant L-683590, respectively (Mirjalili *et al*., 1999; Choi *et al*., 1998; Junker *et al*., 1998).

From a screen of different oils, methyl oleate was identified as the leading candidate in promoting cell growth and epothilones D production. These improvements, however, were observed only with the simultaneous addition of trace metals to the production medium. The sole addition of methyl oleate at 7 mL/L increased the maximum cell density from 0.4 g DCW/L to 1.2 g DCW/L, but the production remained at the baseline leveL With the exogenous supplement of 4 mL/L of trace elements, the peak biomass concentration increased to 2.1 g DCW/L, and the epothilone D titer was boosted from 0.45 mg/L to 3.3 mg/L. These findings indicate that nutritional components deficient in casitone may be important for the growth of *M. xanthus* and the formation of epothilones.

With the establishment of optimal concentrations of methyl oleate and trace elements for a batch process, efforts were made to develop a feeding strategy to maintain vigorous cell growth and to prolong the production period. Intermittent and continuous feeds of casitone and methyl oleate at constant rates were evaluated, and both methods resulted in similar improvements in the growth profiles. With optimal feeds of the two substrates, maximum cell densities of about 6.8 g DCW/L were obtained. In both cases, methyl oleate was depleted as it was added to the fermentation

medium.

In contrast to cell growth and methyl oleate consumption, epothilone production and ammonia generation were greatly influenced by the choice of the feeding strategy. In the continuous fed-batch culture, a peak epothilone D titer of 23 mg/L was obtained. This was nearly 2.5 times the titer obtained for the intermittent fed-batch culture. With the continuous feeds, the rate at which ammonia was released by the cells was also twice as high, suggesting a higher rate of casitone consumption. Together, these results indicate that the lower titers associated with the intermittent fed-batch process may have been caused by catabolite repression and not ammonia accumulation. Moreover, they suggest that the productivity of the *M. xanthus* strain is sensitive to the amount of substrates present in the culture medium and may be maximal under substrate-limiting conditions. This is also consistent with the observation that increasing casitone concentration in the production medium results in higher cell densities but lower titers.

Compared to the batch cultures with the basal medium, the continuous fed-batch cultures yielded a 17-fold increase in cell density and a 140-fold increase in titer. This process has been scaled from 5-L to 1000-L and shown to perform equivalently. The results shown for producing epothilone D on a manufacturing scale demonstrate that *M. xanthus* can be used as a host for the production of other biologically active molecules from myxobacteria.

*REFERENCES*


Bollag et al. 1995. Epothilones, a new class of microtubule-stabilizing agents with a taxol-like mechanism of action. Cancer Res 55:2325-2333.

Choi et al. 1998. Effects of rapeseed oil on activity of methylmalonyl-CoA carboxyltransferase in culture of Streptomyces fradiae. Biosci Biotechnol Biochem 62902 906.

Chou et al. 1998. Desoxyepothilone B: An efficacious miczotubule-targeted antitumor agent with a promising in vivo profile relative to epothilone B. Proc Natl Acad Sci USA 95:9642-9647.

Gerth et al. 1996. Epothilons A and B: Antifungal and cytotoxic compounds from Sorangium cellulosum (myxobacteria) - production, physico-chemical, an biological properties. J Antibiot (Tokyo) 49:560-563.

Gerth et al. 2000. Studies on the biosynthesis of epothilones: the biosynthetic origin of the carbon skeleton. J Antibiot (Tokyo) 53:1373-1377.

Gerth et al. 2001. Studies on the biosynthesis of epothilones: The PKS and epothilone C/D monooxygenase. J Antibiot (Tokyo) 54:144-148.

Harris et al. 1999. New chemical synthesis of the promising cancer chemotherapeutic agent 12,13-desoxyepothilone B: Discovery of a surprising long-range effect on the diastereoselectivity of an aldol condensation. J Am Chem Soc 12:7050-7062.

Hecht et al. 1990. Hollow fiber supported gas membrane for in situ removal of ammonium during an antibiotic fermentation. Biotechnol Bioeng 35:1042-1050.

Junker et al. 1998. Use of soybean oil and ammonium sulfate additions to optimize secondary metabolite production. Biotechnol Bioeng 60:580-588.

Kowalski et al. 1997. Activities of the microtubule-stabilizing agents epothilones A and B with purified tubulin and in cells resistant to paclitaxel. J Biol Chem 272:2534-41.

Meng et al. 1997. Remote effects in macrolide formation through ring-forming olefin metathesis: An application to the synthesis of fully active epothilone congeners. J Am Chem Soc 119:2733-2734.

Mirjalili et al. 1999. The effect of rapeseed oil uptake on the production of erythromycin and triketide lactone by Saccharopolyspora erythraea. Biotechnol Prog 15:911-918.

Molnár et al. 2000. The biosynthetic gene cluster for the microtubule-stabilizing agents epothilones A and B from Sorangium cellulosum So ce90. Chem Biol 7:97-109.

Reichenbach et al. The Prokaryotes II Eds. New York: Springer-Verlag. p 3417-3487.

Reichenbach et al. 1993. Production of Bioactive Secondary Metabolites. In: Dworkin M, Kaiser D, editor. Myxobacteria II. Washington, DC: American Society for Microbiology. p 347-397.

Sinha et al. 1998. The antibody catalysis route to the total synthesis of epothilones. Proc Natl Acad Sci USA 95:14603-14608.

Su et al. 1997. Structure-activity relationships of the epothilones and the first in vivo comparison with paclitaxel. Angew Chem Int Ed Engl 36:2093-2096.

Tang et al. 2000. Cloning and heterologous expression of the epothilone gene cluster. Science 287:640-642.


<u>C. Production of Epothilone D</u>

*Flasks*

A 1 mL vial of the K111-40-1 strain (described in Example 4) is thawed and the contents transferred into 3 mL

of CYE seed media in a glass tube. This culture is incubated for 72±12 hours at 30°C, followed by the subculturing of 3 mL of this tube culture into 50 mL of CYE media within a 250 mL baffled Erlenmeyer flask. This CYE flask is incubated for 24±8 hours at 30°C, and 2.5 mL of this seed (5 % v/v) used to inoculate the epothilone production flasks (50 mL of 1x wheat gluten media in a 250 mL baffled Erlenmeyer flask). These flasks are then incubated at 30°C for 48±12 hours, with a media pH at the beginning of 7.4.

*Fermentors*

A similar inoculum expansion of K111-40-1 as described above is used, with the additional step that 25 mL of the 50 mL CYE seed is subcultured into 500 mL of CYE. 250 mL of this secondary seed is used to inoculate a 5 L fermentor containing 4.5 L of CT5-TA, with a 1 g/L daily feed of sodium pyruvate. The process parameter setpoints for this fermentation are: pH - 7.4; agitation - 400 rpm; sparge rate - 0.15 vvm. These parameters were sufficient to maintain the DO at greater than 80% of saturation. The pH control is provided by addition of 2.5 N sulfuric acid and sodium hydroxide to the cultures. Peak epothilone titers are achieved at 36±8 hours. The peak epothilone C titer is 0.5 mg/L, and the peak epothilone D titer is 1.6 mg/L.

Table 5 is a summary of the media that were used and their respective components.

**TABLE 5**

| **CYE Seed Media** | **Component** | **Concentration** |
|---|---|---|
| | Casitone (Difco) | 10 g/L |
| | Yeast Extract (Difco) | 5 g/L |
| | $MgSO_4 \cdot 7H_20$ (EM Science) | 1 g/L |
| | HEPES buffer | 50 mM |
| **CTS-TA Production Media** | **Component** | **Concentration** |
| | Casitone (Difco) | 5 g/L |
| | $MgSO_4 \cdot 7H_20$ (EM Science) | 2 g/L |
| | L-alanine, L-serine, glycine | 1 mg/L |
| | HEPES buffer | 50 mM |
| **1x Wheat Gluten Production Media** | **Component** | **Concentration** |
| | Wheat Gluten (Sigma) | 5 g/L |
| | $MgSO_4 \cdot 7H_20$ (EM Science) | 2 g/L |
| | HEPES buffer | 50 mM |

The CYE seed media and the CTS-TA production media are sterizlied by autoSterilized autoclaving for 30 minutes at 121°C. The wheat gluten production media is sterilized by autoclaving for 45 minutes at at 121 °C.

D. Production of Epothilone C and D from *Myxococcus xanthus*

A fermentation process may be used for *Myxococcus* strains, including but not limited to *M. xanthus* K111-40-1, in which the fermentation media provides carbon sources that can be utilized without generation of ammonia. The carbon source may be an oil, such as methyl oleate or a similar oil. In shake flask tests with a variety of feed ratios, these methods resulted in the production of epothilones C and D, predominantly epothilone D, at levels ranging from 15 to 25 mg/L, as described below.

*Seed Culture*

A frozen 1 mL vial of *M. xanthus* K111-40-1 cells that had been grown in 50 mL of CYE medium with 2 mL/L methyl oleate was used to inoculate 3 mL of fresh CYE medium with 1 mL/L methyl oleate in a sterile glass tube. The tube was incubated at 30°C in a 250 RPM shaker for 24 hrs. The inoculum in the glass tube was then transferred into a 250 mL unbaffled flask that contained 50 mL of fresh CYE medium with 2 mL/L methyl oleate. The flask was incubated at 30°C in a 250 RPM shaker for 48 hrs.

*Production Flask*

1 g of Amberlite XAD-16 was sterilized in a 250-mL unbaffled flask by autoclaving at 121°C for 30 min. 50 mL of sterile production media were then added to the flask. The flask was inoculated with 5% (v/v) of the seed culture and was placed in an incubator shaker operating at 250 RPM and 30°C. A 3 mL/L/ day feed of sterile methyl oleate was initiated two days after the time of inoculation, and a 2 g/L/day feed of casitone was initiated one day after the time of inoculation.

*Product Extraction*

After 14 days, the XAD resin in the production flask was transferred into a 50 mL centrifuge tube. Excess medium in the tube was decanted without removing any of the resin. The XAD resin was then washed with 25 mL of water and allowed to settle. The water in the tube was decanted without removing any of the resin, and 20 mL of methanol were added to the tube. The centrifuge tube was placed on a shaker at 175 RPM for 20-30 min. to extract the epothilone products from the resin. The methanol extract was transferred to a new centrifuge tube for storage and LC/MS analysis.

Table 6 is a summary of the media that were used and their respective components.

**TABLE 6**

| **CYE Media** | **Component** | **Concentration** |
|---|---|---|
| | Casitone (Difco) | 10 g/L |
| | Yeast Extract (Difco) | 5 g/L |
| | $MgSO_4 \cdot 7H_20$ (EM Science) | 1 g/L |
| **Production Media** | **Component** | **Concentration** |
| | Casitone (Difco) | 5 g/L |
| | $MgSO_4 \cdot 7H_20$ (EM Science) | 2 g/L |
| Added to Production Media after autoclaving | | |
| | 1000x Trace Element Solution | 4 mL/L |
| | Methyl Oleate | 2 mL/L |

The CYE media and the production media are sterizlied by autoSterilized autoclaving for 30 minutes at 121°C The trace element solution is filter-sterilized; the methyl oleate is autoclaved separately.

Trace element solution is made by combining all of the components in Table 7, adding 10 mL/L concentrated $H_2SO_4$ to the solution and brining the final volume to 1 L.

**TABLE 7**

| **1000x Trace Element Solution** | **Component** | **Concentration** |
|---|---|---|
| | $FeCl_3$ | 8.6 g/L |
| | $ZaCl_2$ | 2.0 g/L |
| | $MnCl_2 \cdot 4H_2O$ | 1.0 g/L |
| | $GuCl_2 \cdot 2H_2O$ | 0.43 g/L |
| | $H_3BO_3$ | 0.31 g/L |
| | $CaCl_2 \cdot 6H_2O$ | 0.24 g/L |
| | $Na_2MoO_4 \cdot 2H_2O$ | 0.24 g/L |

The resulting solution is filtered sterilized.

E. Fermentation, Production, and Purification of Epothilones from Myxoc*occus xanthus*

*Description of M. xanthus strains*

Strain K111-25-1 is the epothilone B producing strain, which also produces epothilone A. Strain K111-40-1 is the epothilone D producing strain, which also produces epothilone C.

*Maintainance of* M. *xanthus on plates*

The *M. xanthus* strains are maintained on CYE agar plates (see Table 8 for plate composition). Colonies appear approximately 3 days after streaking out on the plates. Plates are incubated at 32°C for the desired level of growth and then stored at room temperature for up to 3 weeks (storage at 4°C on plates can kill the cells).

**TABLE 8**

| CYE Agar Plates | Component | Concentration |
|---|---|---|
| | Hydrolyzed casein (pancreatic digest) | 10 g/L |
| | Yeast extract | 5 g/L |
| | Agar | 15 g/L |
| | $MgSO_4$ | 1 g/L |
| | 1 M MOPS buffer solution (pH 7.6) | 10 mL/L |
| *1 L agar media batches are autoclaved for 45 minutes, then poured out into petri dishes. | | |

*Oil adaptation of M. xanthus for Cell Banking*

Transfer a non-oil adapted colony from a CYE plate or a frozen vial of cells into a 50 mL glass culture tube containing 3 mL of CYE seed media and 1 drop of methyl oleate from a 100 μL pipet Allow cells to grow for 2-6 days (30°C, 175 rpm) until the culture appears dense under a microscope. Start several (5 - 7) tubes in parallel, as these cells do not always adapt well to the oil.

*Cell banking procedure (Master Cell Bank*)

Start an oil-adapted tube culture as described above. When the tube culture is sufficiently dense (OD = 5 +/- 1), transfer the entire contents of the tube into a sterile 250 mL shake flask containing 50 mL of CYE-MOM seed media (see table below for media composition). After 48 ± 12 hours of growth in a shaker incubator (30°C, 175 rpm), transfer 5 mL of this seed culture into 100 mL of CYE-MOM in a 500 mL shake flask. Allow this culture to grow for 1 day in a shaker incubator (30°C, 175 rpm). Check culture microscopically for appropriate growth and lack of contamination.

Combine 80 mL of this seed culture and 24 mL of sterile 90% glycerol in a sterile 250 mL shake flask. Swirl to thoroughly mix, and aliquot 1 mL of this mixture into 100 sterile, prelabeled cryovials. Slow freeze vials by placing them in a -80°C freezer.

*Cell banking procedure (Working Cell Bank)*

Start a tube culture by thawing one of the master cell bank vials produced as described above at room temperature, then depositing its entire contents into a glass tube containing 3 mL of CYE-MOM seed media. When this tube culture is sufficiently dense (OD = 5 +/-1), transfer the entire contents of the tube into a sterile 250 mL shake flask containing 50 mL of CYE-MOM seed media. After 48 ± 12 hours of growth (at 30°C, 175 rpm), transfer 5 mL of this seed culture into 100 mL of CYE-MOM in a 500 mL shake flask. Allow this to grow for 1 day (30°C, 175 rpm). Check microscopically for growth and contamination.

Combine 80 mL of this seed culture and 24 mL of sterile 90% glycerol in a sterile 250 mL shake flask Swirl to thoroughly mix, and aliquot 1 mL of this mixture into 100 sterile, prelabeled cryovials. Slow freeze vials by placing them in a -80°C freezer.

*Composition of Seed Media*

The same seed media as described by Table 9 is used for cell banking and the expansion of the cell bank vials up to any required volume.

**TABLE 9**

| CYE-MOM Seed Medium* | Component | Concentration |
|---|---|---|
| | Hydrolyzed casein (pancreatic digest) - Difco | 10 g/L |
| | Yeast extract - Difco | 5 g/L |
| | $MgSO_4 \cdot 7H_2O$ - EM Science | 1 g/L |
| | Methyl Oleate - Cognis | 2 mL/L |
| *Note: the methyl oleate is added after the other ingredients, as it forms an emulsion in the casitone and does not completely mix with the other components. | | |

*Inocula Scaleup for Shake Flask, 5L, and 1000L Fermentations*

Thaw a frozen working cell bank vial of the methyl oleate adapted cells. Transfer the entire contents of the vial into a 50 mL glass culture tube, containing 3 mL of the CYE-MOM seed media. Place tube in a shaker (30°C, 175 rpm), and grow for 48 ± 24 hours. Transfer the entire contents of the culture tube into a 250 mL shake flask containing 50 mL of CYE-MOM seed media. Place flask in a shaker (30°C, 175 rpm) and grow for 48 ± 24 hours. For use in shake flask experiments, expand this seed by subculturing 10 mL of this culture into 40 mL of fresh CYE-MOM in 5 new seed flasks. Incubate seed flasks in shaker (30°C, 175 rpm) for 24 ± 12 hours for use as an inoculum for flask volume (30 -100 mL) production cultures. Inoculate production flasks at 4.5% of the combined (seed and production media) initial volume.

To prepare seeds for small scale (5 -10 L) fermentations, subculture the entire contents of one of these 50 mL seed flasks into a sterile 28 L fernbach flask containing 500 mL of CYE-MOM. Incubate this fernbach flask in a shaker (30°C, 175 rpm) for 48 ± 24 hours for use as the fermentor inoculum. Inoculate the production fermentation at about 5% of the combined initial volume.

Further seed expansion is required for large scale (1000 L) fermentations. Here, 1 L of the fernbach flask seed is used to inoculate (5% by volume) a 10 L seed fermentor containing 9 L of CYE-MOM. The fermentor pH is controlled at 7.4 by addition of 2.5 N potassium hydroxide and 25 N sulfuric acid. The temperature is set at 30°C. The dissolved oxygen is maintained at or above 50% of saturation by cascading of the stir rate between 400 - 700 rpm. The initial agitation rate is set at 400 rpm, and the sparging rate was maintained at 0.1 v/v/m. After 24 ± 12 hours of growth in the 10L fermentor, the entire culture is transferred into a 150 L fermentor containing 90 L CYE-MOM. The pH is once again controlled at 7.4 with 2.5 N potassium hydroxide and 2.5 N sulfuric acid. The temperature is set at 30°C. The dissolved oxygen is maintained at or above 50% of saturation by cascading of the stir rate between 400 - 700 rpm. The initial agitation rate is set at 400 rpm, and the sparging rate is maintained at 0.1 v/v/m.

*XAD-16 Resin Preparation for Fermentations*

Transfer the required amount of XAD-16 resin (Rohm & Haas) into a methanol safe container with a minimum volume of 3 times the weight of XAD-16 resin (*i.e.*, 1.2 kg of resin requires a container of at least 3.6 L). Wash the resin thoroughly with 100% methanol to remove any monomers present on the virgin resin. Add two times the amount of methanol in liters as the weight of the resin in kilograms (*i.e.* 6 liters of methanol for 3 kilograms of XAD-16). Mix the methanol and XAD slurry for 5 minutes to remove any monomers present on the XAD-16. Stir the slurry gently while mixing to minimize resin fragmentation. Stop mixing, and allow the resin to gravity settle for not less than 15 minutes. Drain the methanol from the container, leaving a 0.5 to 1 inch layer of methanol above the XAD bed. Transfer the XAD and methanol from the mixing container to an Amicon A250 column. Attach the top bed support to the column and seal the bed support by turning the seal adjust knob clockwise. Wash the XAD in the column with not less than 5 column volumes of methanol at 300 ± 50 cm/hr. Collect methanol flow through in the solvent waste receptacle. Wash the XAD in the column with not less than 10 column volumes of deionized water at 300 ± 50 cm/hr.

The composition of the epothilone production media is described in Table 10.

**TABLE 10**

| CTS-MOM Production Media | Component | Concentration |
|---|---|---|
| | Casitone (Difco) | 5 g/L |
| | $MgSO_4 \cdot 7H_20$ (EM Science) | 2 g/L |
| | XAD-16 | 20 g/L |
| Added after autoclaving | | |
| | 1000x Trace Element Solution | 4 mL/L |
| | Methyl Oleate | 2 mL/L |
| ****Note:** the methyl oleate is added after the other ingredients, as it forms an emulsion in the casitone and does not completely mix with the other components. Trace Element Solution is as described in Table 7.* | | |

*Preparation and Flosk-scale (50* mL) *Epothilone Production Fermentation*

Autoclave 1g of XAD-16 in a 250 mL shake flask with sufficient deionized water (~ 3 mL) to cover the resin Flasks are sterilized by autoclaving for 30 minutes at 121°C. Add the following media components to the flask aseptically: 50 mL of CTS-MOM production medium, and 2.5 mL of 1 M HEPES buffer (titrated to a pH of 7.6 with potassium hydroxide). Inoculate the cultures with 2.5 mL of the CYE seed flask (4.5% volume/volume inoculum). Incubate the production flasks on a shaker at 30°C and 175 rpm.

Start the casitone and methyl oleate feeds 24 ± 6 hours after inoculation At this point, and every 24 ± 6 hours thereafter, feed 1 mL of a 100 g/L casitone solution and 150 μL of methyl oleate. Continue this feeding regimen for up to 13 days following the initial feed, or until cells are observed to begin lysis (day 11-14). To determine epothilone production kinetics, a representative 5 mL sample of well-mixed fermentation broth and XAD can be sampled. Additionally, a small (0.25 - 0.5 mL) sample of broth without the XAD can be taken daily to check on the status of the culture growth visually. When massive cell lysis is observed, the remainder of the culture volume should be harvested.

*Preparation and 5 L-scale Epothilone Production Fermentation*

100 g XAD and 8 g $MgSO_4$-$7H_2O$ are combined with 3.9 L of deionized water, and sterilized (90 minutes, 121°C) in a 5 L B-Braun bioreactor. A sufficient volume (133 mL) of a presterilized casitone / deionized water solution (150 g/L) is pumped in aseptically to attain a final casitone concentration of 5 g/L in the fermentor. An initial methyl oleate concentration of 2 mL / L is achieved by addition of 10 mL of this oil. Finally, 16 mL of a presterilized trace elements solution are added aseptically prior to inoculation The fermentor is then inoculated with 200 mL of the CYE seed culture (4.8% volume / volume) and permitted to grow for 24 ± 6 hours. At this point, the casitone (2 g / L / day, continuous feed) and methyl oleate (3 mL / L / day total, fed semi-continuously every 90 minutes) feeds are initiated. Airflow is held constant in the bioreactor at 0.4 - 0.5 vvm (the increasing fermentation volume as the feeds progress causes this variation). The dissolved oxygen concentration is controlled at 50% of saturation by a stirring cascade (400-700 rpm). The 100% of saturation dissolved oxygen calibration point is established by setting the initial agitation at 400 rpm, and the initial airflow at 0.5 vvm. The pH setpoint of 7.4 is maintained by automated addition of 2.5N $H_2SO_4$ and 25N KOH. Epothilone production continues for 11-14 days following inoculation, with the bioreactor is harvested when cell lysis is apparent in the broth samples and the demand for oxygen (as indicated by the agitation rate) abruptly decreases. Epothilone D titers generally reach 18 - 25 mg / L in this fermentation process.

*Preparation and 1000 L-scale Epothilone Production Fermentation*

The 1000 L fermentor was prepared for epothilone production as follows. 600 L of water and 18 L (11.574 kg) of XAD-16 was sterilized (45 minutes, 121°C) in the fermentor. Trace metals and $MgSO_4$ were filter sterilized (through a presterilized 0.2 micron polyethersulfone membrane capsule filter) directly into the fermentation vessel. 2.9 L of the trace elements solution as well as a sufficient quantity of a concentrated $MgSO_4$ solution (to 2 g / L final concentration in the fermentor) were added through the same capsule filter. About 200 L of a mixture of 117 g/L casitone and 175 mL/L methyl oleate was sterilized in a 260 L feed tank. About 32 L of this sterile mixture was added to the 1000 L fermentor. Water was filtered into the vessel (through the same capsule filter) to bring final volume to 710 L. Agitation was 100 rpm. Backpressure was maintained at 100 - 300 mbar. When the dissolved oxygen (DO) reached 50% after inoculation, agitation was increased to 150 rpm. When the DO again reached 50%, agitation was increased to 200 rpm.

DO was controlled at 50% of saturation by cascading the airflow (0 Lpm - 240 Lpm). The pH setpoint was maintained at 7.4 by automated addition of 25 M KOH and 25 N $H_2SO_4$. The fermentor was inoculated with 38 L seed from the 150 L fermentor (5% volume / volume).

Addition of 0.570 L/hour of the casitone - methyl oleate feed solution began after the DO reached 50% (for the second time, about 10 $\pm$ 5 hours after inoculation) and continued until the fermentor was harvested The bioreactor was harvested 10 days following inoculation. Final epothilone D titers were determined to be about 20 $\pm$ 5 mg/L.

*Fermentation Sampling Procedure*

For kinetic experiments in flasks, 5 - 50 mL of thoroughly mixed broth and XAD resin were sampled with a 25 mL pipet and deposited in a 10 or 50 mL conical tube. For bioreactor samples, a 50 mL sample of the mixed broth and resin was deposited in a 50 mL conical tube. The conical tube was then permitted to sit for 10 minutes to permit the XAD to settle to the bottom of the tube. The broth at this point can be decanted from the XAD resin. If the XAD does not settle, then one can remove the broth using a 10 - 25 mL pipette.

*Methanol Extraction of XAD resin for Epothilone Titer Quantitation*

After the XAD resin has gravity settled to the bottom of the sample tube (as per the sampling procedure), all of the supernatant is transferred to a new 50 mL conical tube. Wash the XAD resin once by adding water back to the 50 mL mark, mix thoroughly by inversion, and let the XAD resin gravity settle again. Decant the aqueous mixture from the tube, without pouring out the XAD. The last few mL of water can be removed by using a 1 mL pipetman with the tip pushed down into the base of the tube. Add methanol to the tube up to the 25 mL point, and cap the tube. Place the conical tube horizontally on a shaker for 30 minutes (at 20 - 30°C) to thoroughly extract all of the epothilone from the XAD resin.

*HPLC Procedure for Epothilone Quantitation*

Analysis of epothilones C and D is carried out using a Hewlett Packard 1090 HPLC with UV detection at 250 nm. The methanol-extracted solution from the XAD resin (50 $\mu$L) was injected across two 4.6 x 10 mm guard columns (Inertsil, C18 OD 53, 5 $\mu$m), and a longer column of the same material for chromatographic separation (4.6 x 150 mm). The method was isocratic with 60% acetonitrile and 40% water over an 18 minute run. With this method, epothilone D eluted at 13 minutes and epothilone C eluted at 10.3 minutes. Standards were prepared using epothilone D purified from fermentation broth.

*Dry Cell Weight Procedure for Growth Curve*

Set the temperature on a Sorvall RC5B centrifuge (with the SH-3000 bucket rotor) to 20°C. Weigh a 50 mL conical tube that has been in the 80°C oven for at least a day. Record the tare weight and fermentation sample identification on the side of the tube. Pour or pipet 40 mL of broth (containing no XAD) into the tared tube. Spin the conical tube at 4700 RPM (4200g) for 30 minutes. After sedimentation, pour off the supernatant, and resuspend the cell pellet in 40 mL of deionized water to wash the pellet Spin the tube again (4200g, 30 minutes). Decant the supernatant, and place tube in an 80°C drying oven for at least 2 days. Weigh tube, and record the final weight on the tube. The dried cell weight (DCW) can then be calculated by the following equation:

$$\text{DCW (g / L)} = (\text{Final tube weight (g)} - \text{Tare tube weight (g)}) / .04\ \text{L}$$

*Determination of Ammonium Ion Concentration*

The ammonia concentration of the fermentation broth is routinely assayed in the epothilone fermentations. One mL of fermentation broth is clarified by centrifugation in a microcentrifuge (5 minutes, 12000 rpm). An ammonia assay kit from Sigma (Catalog #171-UV) is used for quantitation, with the clarified fermentation broth substituted in place of the blood plasma described in the kit protocol. As the linear response range of this colorimetric assay is only 0.01176 - 0.882 mmoles/L, the clarified fermentation samples are typically diluted 20 -100 fold in deionized water to assay ammonium concentrations within this range.

*Determination of Residual Methyl Oleate Concentration*

The amount of residual methyl oleate present in the fermentation broth can be estimated by extracting fermentation broth samples with methanol, and running these extracted broth samples on an HPLC. Quantitation of the methyl oleate concentration was carried out using a Hewlett Packard 1090 HPLC with UV detection at 210 nm. Whole broth samples (1- 4 mL) were extracted with an equivalent volume of methanol and centrifuged at 12,000 g to sediment any insoluble components. The clarified supernatant (50 μL) was injected onto a 4.6 x 10 mm extraction column (Inertsil, C18 OD 53, 5 μm), washed with 50% acetonitrile for 2 minutes, then eluted onto the main column (4.6 x 150 mm, same stationary phase and flow rate) with a 24 minute gradient starting at 50% acetonitrile and ending at 100% acetonitrile. The 100% acetonitrile column flow was maintained for 5 minutes. Due to its heterogeneous nature, the methyl oleate elutes as a number of disparate peaks, instead of as single pure compound. However, approximately 64 - 67% of the total methyl oleate extractables appear in two primary peaks that elute at 25.3 $\pm$ 0.2 and 27.1 $\pm$ 0.2 minutes, respectively. Methyl oleate in methanol extracted fermentation samples can be estimated by quantitating the summed area of these two peaks, then calibrating them against the summed area of these two peaks in methyl oleate standards prepared in a 50% water/ methanol solution.

**Purification and Crystallization of Epothilone D**

The present invention provides a purification process for epothilone D and highly purified preparations of epothlone D, more particularly epothilone D in crystalline form. The advantages of the present process include initial purification steps that require only alcohol (such as methanol) and water; which allows for efficient use of product pools and minimizes the necessity for time-consuming and labor-intensive evaporation steps. The present method requires only a single evaporation step, which requires the evaporation of 1 L of ethanol for every 10 - 15 g of epothilone. In the process, a column packed with synthetic polystyrene-divinylbenzene resin such as HP20SS is used to remove both polar and lipophilic impurities. This column generates an intermediate product that contains 10% epothilones and eliminates the need for liquid/liquid extractions that use either highly flammable or toxic solvents.

Another improvement relates to the use of a C18 resin with a 40 - 60 micron particle distribution, such as Bakerbond C18, that allows the use of low pressure columns and pumps (less than 50 psi), which reduces cost significantly. The starting material for the C18 chromatography step is solution loaded in a dilute loading solvent. The solvent is weak enough so that epothilones stick at the top of the column in a highly concentrated, tight band, which allows the column to perform well under heavy loading (2 - 5 g epothilone / L resin). Because typical column loading is 1 g/l or less, and chromatography is usually the most expensive step in purification, this improvement results in significant cost savings. Moreover, the present method allows for the use of an alcohol, such as methanol, instead of acetonitrile in the chromatography step. The pool containing the epothilone is crystallized from a binary solvent system in which water is the forcing solvent to provide the epothilone in crystalline form.

The purification process, in one embodiment, consists of the following steps and materials. The XAD resin in the fermentation broth is (1) collected in a filter basket, and (2) eluted to provide an XAD extract, which is (3) diluted with water, and (4) passed through an HP20SS column to provide the HP20SS pool. The HP20SS pool is (5) diluted with water, and (6) subjected to C18 chromatography to provide an epothilone pool, which is (7) diluted with water, and (8) subjected to solvent exchange to provide a concentrated epothilone pool. The concentrated epothilone pool is (9) subjected to charcoal filtration, (10) evaporated, and (11) crystallized to provide highly purified material.

A total of 11 g of epothilone D was isolated and purified to a white crystalline powder from two 1000-L *Myxococcus xanthus* fermentation runs (1031001K and 1117001K). The purity of the final product was >959b, and the recovery of epothilone D was 71%.

Table 11 summarizes the HPLC Methods used during purification.

**TABLE 11**

| | | |
|---|---|---|
| **Epo1 Method** | | |
| | Column | Inertsil ODS3, 5 μm, 4.6 x150 mm |
| | Flow rate | 1 ml/min |
| | Column Oven | 50 °C |
| | Run Time | is minutes |
| | Detection | UV at 250 nm |
| | Gradient | 0 min; 60:40 ACN/$H_2$0<br>12 min; 100:0 ACN/$H_2$0<br>12.1 min; 60:40 ACN/$H_2O$ |
| **Epo78 Method** | | |
| | Column | Inertsil ODS3, 5 μm, 4.6 x150 mm |
| | Flow rate | 1 ml/min |
| | Column Oven | 50 °C |
| | Run Time | 5 minutes |
| | Detection | UV at 250 nm |
| | Gradient | 0 min; 78:22 ACN/$H_2$0 |

The Materials used in this section are as follows. HP20SS resin was purchased from Mitsubishi. The C18 resin was purchased from Bakerbond C18 40g and the methanol was puchased from Fisher Bulk (55 gal). Deionized water was used.

**Fermentation Run 1031001K**

*Step 1 XAD Elution (K125-173)*

Seventeen liters (17 L) of XAD-16 resin were filtered from the fermentation culture using a Mainstream filtration unit with a thirteen-liter 150 μm capture basket The captured XAD resin was packed into an Amicon VA250 column and was washed with 65 L (3.8 column volumes) of water at 1.0 L/min. The epothilone D product was then eluted from the resin using 230 L of 80 % methanol in water.

*Step 2 Solid Phase Extraction (K125-175)*

Seventy-seven liters (77 L) of water were added to the step 1 product pool (230 L) to dilute the loading solvent to 60% methanol in water. The resulting suspension (307 L) was mixed and loaded onto an Amicon VA180 column packed with 5 L of HP20SS resin that had previously been equilibrated with 5 column volumes of 60% methanol. The loading flow rate was 1 L/min. After loading, the column was washed with 13 L of 60% methanol and eluted with 77 L of 75% methanol at a flow rate of 325 mL/min. Thirty-one 2.5-L fractions were collected. Fractions 10-26 (42.5 L) were found to contain epothilone D, and these fraction were pooled together.

*Step 3 Chromatography (K125-179)*

The step 2 product pool was evaporated to an oil using two 20-L rotovaps. During evaporation it was necessary to add ethanol in order to minimize foaming. The dried material was re-suspended in 1.0 L of methanol and diluted with 0.67 L of water to make 1.67 L of a 60% methanol solution. The resulting solution was loaded onto a 1-L C18 chromatography column (55 x 4.8 cm) that had previously been equilibrated with 3 column volumes of 60% methanol The loading flow rate averaged at 64 mL/min. The loaded column was washed with one liter of 60% methanol, and elution of the epothilone D product was carried out isocratically using 70% methanol at a flow rate of 33 mL/min. A total of 27 fractions were collected, with the first fraction containing 3.8 L by volume. This was followed by three 500-mL fractions and twenty-three 250-mL fractions. Fractions 5-20 were taken as the best pool (K125-179-D), containing 4.8 g of epothilone D. Fractions 3-4 (K125-179-C) contained 1.4 g of epothilone D. Because this pool also contained high concentrations

of epothilone C, it was set aside for re-work (Step 3b).

*Step 4 Chromatography (K119-153)*

Epothilone D fractions that also contained high concentrations of the C analog were re-chromatographed on C18 resin as follows. A 2.5 x 50 cm column was packed with C18 resin, washed with 1 L of 100% methanol, and equilibrated with 1 L of 55% methanol in water at a flow rate of 20 mL/min. The pressure drop was 125 psi. The starting material (KI25-179-C, 1040 mL) was diluted with 260 mL of water so that the loading solution contained 55% methanol in water. The resulting solution (1300 mL) was loaded onto the resin, and an additional 250 mL of 55% methanol was passed through the column. The column was first eluted with 5 L of 65% methanol, followed by 3 L of 70% methanol in water. During the 65% methanol elution, a total of forty-eight 100-mL fractions were collected. After switching to 70 % methanol, a total of ten 250-mL fractions were collected. The best epothilone D pool (K119-153-D), consisting of Fractions 50-58, contained 1.0 g of the desired product.

*Step 5a Crystallization (K119-158)*

The starting material for this step was a combination of chromatography products from step 3 and 4. Initially, 120 mL of ethanol was added to 7.9 g of solids containing 5.5 g of epothilone D. With gentle mixing, the solids were completely dissolved, and the solution was transferred to a 400-mL beaker that was placed on a stir plate in a fume hood. A 1" stir bar was added, and the solution was rapidly stirred. Meanwhile, 100 mL of water were slowly added over a period of about 5 minutes. When the formation of small white crystals were observed, the solution was stirred for 15 more minutes until the solution became thick with white solids. The beaker was then removed from the stir plate, covered with aluminum foil, and placed in a refrigerator (2°C) for 12 hours. The white solids were filtered using Whatman #50 filter paper, and no additional wash was performed on this first crop. The solids were placed to a crystallization dish and dried in a vacuum oven (40 °C at 15 mbar) for 1 hour. Subsequently, the material was removed from the oven, made into finer particles, and dried in the vacuum oven for another four hours. This crystallization process yielded 3.41 g of off white solids. The Epo1 HPLC method was used to determine the chromatographic purity of the final product The HPLC results, along with the corresponding $^{1}H$ and $^{13}C$ NMR data, all confirmed that the dried material contained >95% epothilone D. The recovery for this first crop was 58%.

*Step 5b Crystallization (K119-167)*

The starting material for this step was the evaporated mother liquor from step 5a Initially, 70 mL of ethanol and 30 mL of water were added to 3.4 g of solids containing 21 g of epothilone D. This clear solution was transferred to a beaker, and 1 g of decolorizing charcoal was added to it. The mixture was stirred on a medium setting for 10 min. and was then filtered using a Whatman #50 filter paper. The charcoal was washed with two 10-mL aliquots of ethanol and was filtered again. The combined filtrate was brought to dryness using a rotovap, and the solids were re-suspended in 50 mL of ethanol. The resulting solution was placed in a 250-mL beaker, and with good stirring, 50 mL of water was slowly added. To promote crystal formation, a small amount of seed crystal (1 mg) was added to the mixture. After several minutes of stirring, the formation of additional white solids was observed. A stream of nitrogen was set to gently blow over the mixture while the stirring continued. After 15 minutes, the beaker was placed in the refrigerator at 2°C for 36 hours. The mixture was filtered using a Whatman #50 filter paper to capture the crystals, and an additional 7 mL of 50:50 ethanol: water was used to wash the solids. The crystals were subsequently dried in the vacuum oven for 4 hours. This crystallization step yielded 1.46 g of white crystals, which contain >95% epothilone D.

**Fermentation Run 1117001K**

*Step 1 XAD Elution (K125-182)*

Seventeen liters (17 L) of XAD-16 resin were filtered from the fermentation culture using a Mainstream filtration unit with a thirteen-liter 150 μm capture basket The captured XAD resin was packed into an Amicon VA250 column and was washed with 58 L (3.4 column volumes) of water at 1.0 L/min. The epothilone D product was then eluted from the resin using 170 L of 80% methanol in water. During the water wash and the first column volume of elution, the column backpressure increased steadily to above 3 bars with a final flow rate of under 300 mL/min. Therefore, the XAD resin was removed from the column and repacked into an alternate Amicon VA250 column. After the exchange, the backpressure decreased below 1 bar and the flow rate was maintained at 1.0 L/min. A single 170-L fraction was collected in a 600-L stainless steel tank. Based on HPLC analysis, the step 1 product pool was found to contain 8.4 g of epothilone D.

*Step 2 Solid Phase Extraction (K145-150)*

Fifty-seven liters (57 L) of water were added to the step 1 product pool (170 L) to dilute the loading solvent to 60% methanol in water. The resulting suspension (227 L) was stirred with an overhead lightning mixer and loaded onto an Amicon VA180 column packed with 6.5-L of HP20SS resin that had previously been equilibrated with 5 column volumes of 60 % methanol. The loading flow rate was 1 L/min. After loading, the column was washed with 16 L of 60% methanol and eluted with 84 L of 75% methanol at a flow rate of 300 mL/min. Seven fractions were collected with volumes of 18 L, 6 L, 6 L, 6 L, 36 L, 6 L, and 6 L, respectively. Fractions 4 and 5, which contained a total of 8.8 g of epothilone D, were pooled together.

*Step 3 Chromatography (K145-160)*

The step 2 product pool was evaporated to an oil using two 20-L rotovaps. To minimize foaming during the evaporation process, 10 L of ethanol were added to the mixture. The dried material was resuspended in 28 L of methanol and diluted with 3.4 L of water to make 6.2 L of a 45% methanol solution. The resulting solution was pumped onto a 1-L C18 chromatography column (55 x 4.8 cm) that had previously been equilibrated with 5 column volumes of 45% methanol. The loading flow rate averaged at 100 mL/min. The loaded column was washed with one liter of 60 % methanol, and the epothilone D product was eluted from the resin using a step gradient at a flow rate of 100 mL/min. The column was eluted with 5 L of 55% methanol, 11.5 L of 60% methanol, and 13.5 L of 65% methanol. During the 55% methanol elution, a total of ten 500-mL fractions were collected. After switching to 60% methanol, a total of twenty-three 500-mL fractions were collected. During the final 65% methanol elution, eleven 500-mL fractions were collected, followed by eight 1-L fractions. The best epothilone D pool (K145-160-D), consisting of Fractions 28-50, contained 8.3 g of the desired product Fractions 26-27 (K145-160-C), which were contaminated with 0.4 g of the epothilone C, contained 0.2 g of epothilone D. All of these 25 fractions were combined.

To dilute product pool to 40% methanol in water, 9.5 L of water was added to 15.8 L of the loading solution. The resulting solution (25.3 L) was then pumped onto a 700-mL C18 chromatography column (9x10 cm) that had previously been equilibrated with 4 column volumes of 40% methanol. The loading flow rate averaged at 360 mL/min. The loaded column was washed with one liter of 40% methanol, and the epothilone D product was eluted from the resin with 3.75 L of 100% ethanol. The eluant was evaporated to dryness using a rotovap. The solids were resuspended in 100 mL of acetone, and the undissolved material was filtered from the solution using a Whatman #2 filter paper. The filtered particles were washed with an additional 115 mL of acetone and filtered once more. Following the acetone extraction, 2 g of decolorizing charcoal were added to the combined filtrate. The mixture was stirred on a medium setting for 1 hour and was filtered using a Whatman #50 filter paper. The charcoal was washed with 180 mL of ethanol and was filtered again. The filtrates were pooled together and rotovaped to dryness.

*Step 4 Chromatography (K119-174)*

The dried material from step 3 was resuspended in 5.0 L of 50% methanol in water and was loaded onto a 1-L C18 chromatography column (55 x 4.8 cm) that had previously been equilibrated with 3 column volumes of 50% methanol. The loading flow rate averaged at 80 mL/min. The column was subsequently washed with one liter of 50% methanol, and the epothilone D product was eluted isocratically from the resin using 70% methanol at the same flow rate. A total of 48 fractions were collected, with the first 47 fractions containing 240 mL and the last fraction containing 1 L. Fractions 25-48 were taken as the best pool (K119-174-D), containing 7.4 g of epothilone D. Fractions 21-24 (K119-174-C) contained 1.1 g of epothilone D. Because this pool also contained high concentrations of epothilone C, it was set aside for re-work.

*Step 5 Crystallization (K119-177)*

To perform a solvent exchange prior to the crystallization step, 3.9 L of water was added to 6.4 L of the best epothilone D pool (K119-174-D) from step 5 to dilute the loading solution to 40% methanol in water. The resulting solution was then loaded onto a 200-mL C18 chromatography column (2.5 x 10 cm) that had previously been equilibrated with 3 column volumes of 40% methanol. The loaded column was washed with 200 mL of 40.% methanol, and the epothilone D product was eluted from the resin with 1 L of 100% ethanol. The eluant was evaporated to dryness using a rotovap, and the solids were re-suspended with 150 mL of 100% ethanol. The clear solution was transferred to a beaker and with good stirring, 175 mL of water was slowly added. A small (1 mg) seed crystal was also added to the solution to promote crystal formation. When the formation of small white crystals were observed, the solution was stirred for 15 more minutes until the solution became thick with white solids. The beaker was then removed from the stir plate, covered with aluminum foil, and placed in a refrigerator (2°C) for 12 hours. The white solids were filtered using Whatman #50

filter paper, and no additional wash was performed on this first crop. The solids were placed to a crystallization dish and dried in a vacuum oven (40°C at 15 mbar) for 6 hours. This crystallization process yielded 6.2 g of white solids, which contained >95% epothilone D. The recovery for this first crop was 74%.

*Results*

The epothilone D recovery for run 1031001K was 4.8 g of crystalline material at a purity of about 97.5 - 98.8%. The epothilone D recovery for run 1117001K was 6.2 g of crystalline material at a purity of about 97.7%. The impurity profiles for these runs are shown in Table 12.

**TABLE 12**

| 1031001K run | Step | Product | Epo C | Epo490 | Epo D |
|---|---|---|---|---|---|
| | 2 | SPE | 23 | 4 | 74 |
| | 3-4 | Total Chrom | 0.7 | 0.7 | 90.6 |
| | 5a | Crystallization | 1.0 | 1.0 | 97.5 |
| | 5b | Crystallization | 0.7 | 0.5 | 98.8 |
| **1117001K run** | **Step** | **Product** | **Epo C** | **Epo490** | **Epo D** |
| | 2 | SPE | 18 | 2 | 60 |
| | 3 | C18 Chrom | 52 | 1.6 | 81.4 |
| | 4 | C18 Chrom | 1.6 | 1.8 | 96.6 |
| | 5 | Crystallization | 0.8 | 1.4 | 97.7 |

"Epo490" is a novel epothilone compound of the invention, 10,11-dehydro-epothilone D, that is produced by the *Myxococcus* host cells.

This purification methodology arose out of efforts to scale-up modifications made to the epothilone D purification process to accommodate the use of methyl oleate in the fermentation medium. The elution of the epothilone D product from the XAD resin was carried out in a straightforward manner. Instead of using 100% methanol, 10 column volumes of 80% methanol were used to elute the product from the beads in a column. During the XAD elution step, it was noted that the presence of lysed cells in the fermentation broth may contribute to the clogging of the purification columns. The harvest of the 103100-1K fermentation run had occurred before significant cell lysis had taken place, while the 111700-1K fermentation run was harvested only after considerable cell lysis had occurred. However, a high backpressure and a low flow rate were observed only for the latter run during the elution process. Therefore, it is likely that the lysed cells in this run may have aggregated and subsequently fouled the column filter.

These purification runs show that epothilone D is stable at room temperature in 80% methanol for at least one day. Based on HPLC analysis, degradation of the product under these conditions is not detectable. This finding allowed storage of the 170-L product pool from the XAD elution step in a 600-L stainless steel tank overnight without refrigeration. To further improve the process, a solvent-exchange column was employed, which is much less time-consuming and labor-intensive than the use of a rotovap in concentrating the volume of the product pools. Therefore, one can replace large-scale rotovaping with a solvent-exchange step.

Although a significant amount oil remained bound to the resin during the XAD elution step, a sizable amount was still present in the eluant Even after the HP20SS solid phase extraction, oil droplets were clearly visible in the product pool and proved to problematic during the C18 chromatography. For optimal chromatography performance, the concentration of epothilone D in the loading solution should be kept below 2 g/L. At higher concentrations, the starting material has a tendency to oil out on the column.

Crystallization was not possible when feed material contained more than 3% of either epothilone C or epo490. This was the case during the purification of 1117001K The first chromatography step gave a product that contained 5% epothilone C. After numerous attempts, crystallization of this material was not achieved. However, taking this material through a second chromatography step reduced epothilone C to 1% and generated a feed material that was easily crystallized.

Example 4

Construction of a *Myxococcus* Strain with a Non-functional *epoK* Gene

Strain K111-40-1 was constructed from strain K111-32.25 by insertional inactivation of the *epoK* gene. To construct this *epoK* mutant, a kanamycin resistance cassette was inserted into the *epoK* gene. This was done by isolating the 4879 bp fragment from pKOS35-79.85, which contains *epoK*, and ligating it into the *Not*I site of pBluescriptSKII+. This plasmid, pKOS35-83.5, was partially cleaved with *Sca*I, and the 7.4 kb fragment was ligated with the 1.5 kb *Eco*RI-*Bam*HI fragment containing the kanamycin resistance gene from pBJ180-2, which had the DNA ends made blunt with the Klenow frangment of DNA polymerase I, to yield plasmid pKOS90-55. Finally, the ~400 bp RP4 oriT fragment from pBJ183 was ligated into the *Xba*I and EcoRI sites to create pKOS90-63. This plasmid was linearized with *Dra*I and electroporated into the *Myxococcus xanthus* strain K111-32.25, and transformants selected to provide *M. xanthus* strain K111-40.1. Strain K111-40.1 was deposited in compliance with the Budapest Treaty with the American Type Culture Collection, Manassas, VA 20110-2209, USA on Nov. 21, 2000, and is available under accession No. PTA-2712

To create a markerless *epoK* mutation, pKOS35-83.5 was cleaved with *Sau*I and the 2.9 kb and 4.3 kb fragments were ligated together. This plasmid, pKOS90-101, has an in-frame deletion in *epoK.* Next, the 3 kb *Bam*HI and *Nde*I fragment from KG2, which had the DNA ends made blunt with the Klenow fragment of polymerase I and contains the kanamycin resistance and *galK* genes, was ligated into the *Dra*I site of pKOS90-101 to create pKOS90-105. This plasmid was electroporated into K111-32.25 and kanamycin resistant electroporants were selected. To replace the wild type copy of *epoK* with the deletion, the second recombination event was selected by growth on galactose plates. These galactose resistant colonies are tested for production of epothilone C and D, and a producing strain was designated K111-72.4.4 and deposited in compliance with the Budapest Treaty with the American Type Culture Collection, Manassas, VA 20110-2209, USA on Nov. 21, 2000, and is available under accession No. PTA-2713.

## Claims

1. Crystalline epothilone D.

2. A method for obtaining epothilone D in crystalline form comprising crystallizing epothilone D from a binary solvent system in which water is the forcing solvent.

3. A method according to claim 2, wherein the binary solvent system is ethanol and water.

4. A method according to claim 3, wherein the epothilone D in crystalline form is more than 95% pure.

## Patentansprüche

1. Kristallines Epothilon D.

2. Verfahren zur Erhaltung von Epothilon D in kristalliner Form umfassend das Kristallisieren von Epothilon D von einem binären Lösungsmittelsystem in welchem Wasser das Zwangslösungsmittel ist.

3. Verfahren nach Anspruch 2, wobei das binäre Lösungsmittelsystem Ethanol und Wasser sind.

4. Verfahren nach Anspruch 3, wobei das Epothilon D in kristalliner Form zu mehr als 95 % pur ist.

## Revendications

1. Epothilone D cristalline.

2. Procédé pour l'obtention d'épothilone D sous forme cristalline comprenant la cristallisation de l'épothilone D à partir d'un système de solvant binaire dans lequel l'eau est le solvant de forçage.

3. Procédé selon la revendication 2, dans lequel le système de solvant binaire sont l'éthanol et l'eau.

4. Procédé selon la revendication 3, dans lequel l'épothilone D sous forme cristalline est pure à plus de 95 %.

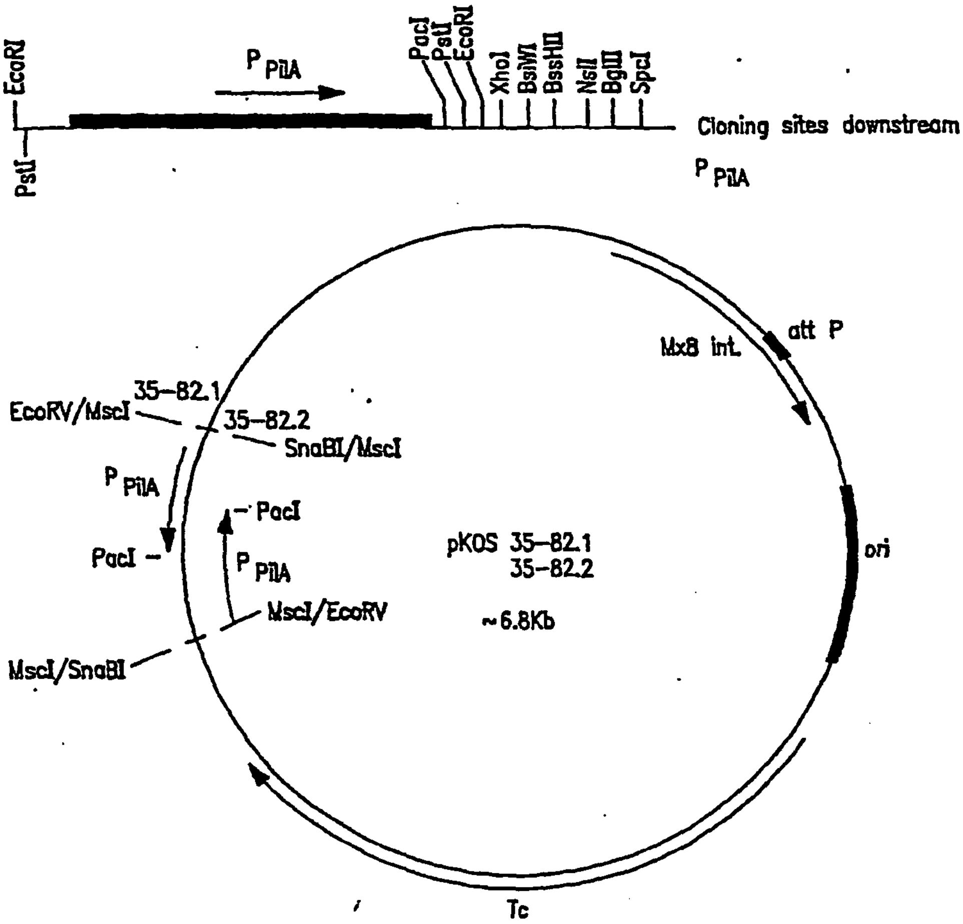
EcoRI
PstI
P PilA
PacI
PstI
EcoRI
XhoI
BsiWI
BssHII
NsiI
BglII
SpcI
Cloning sites downstream
P PilA
att P
Mx8 int.
ori
Tc
35-82.1
EcoRV/MscI
35-82.2
SnaBI/MscI
P PilA
PacI
PacI
P PilA
MscI/EcoRV
MscI/SnaBI
pKOS 35-82.1
35-82.2
~6.8Kb

# FIGURE 1

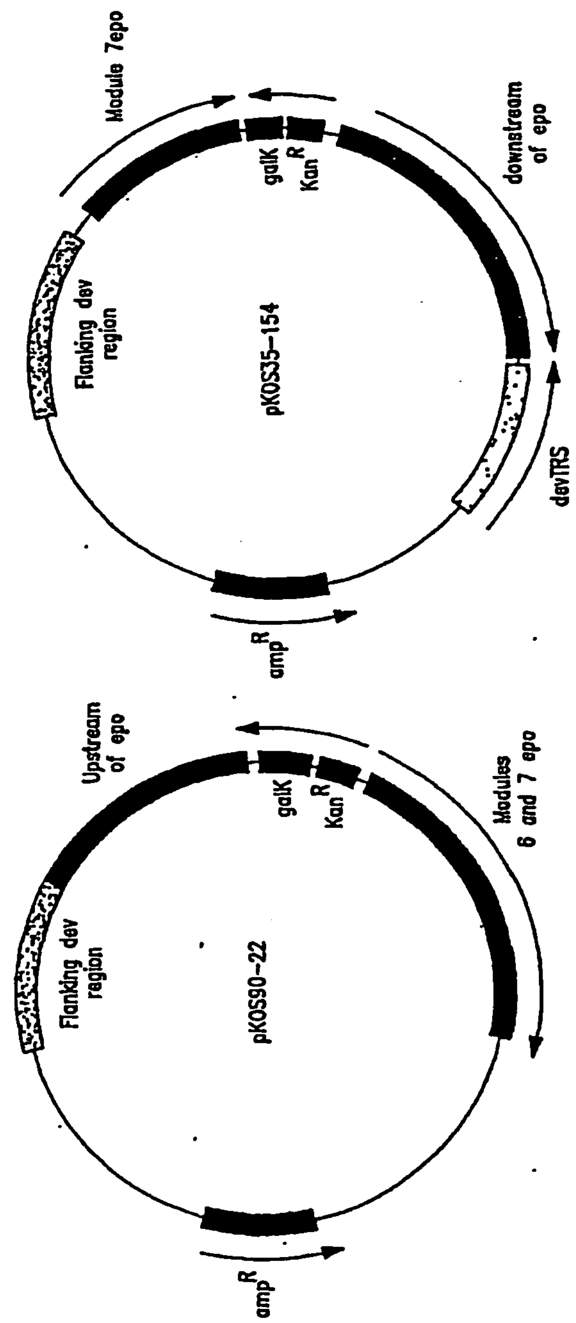
pKOS35-154
Module 7epo
galK
Kan^R
downstream of epo
Flanking dev region
devTRS
amp^R
pKOS90-22
Upstream of epo
galK
Kan^R
Modules 6 and 7 epo
Flanking dev region
amp^R

FIGURE 2

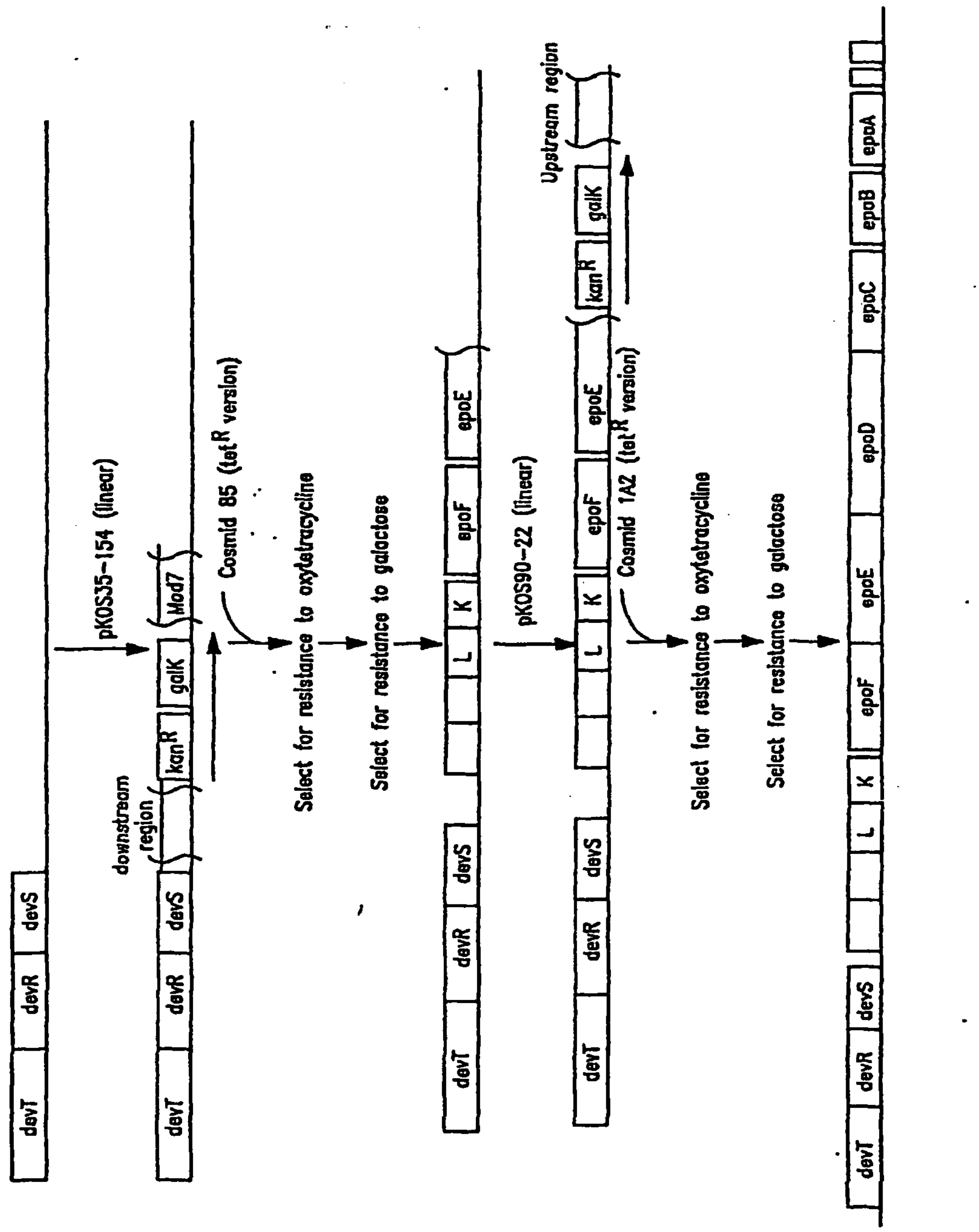
devT
devR
devS
pKOS35-154 (linear)
downstream region
kanR
galK
Mod7
Cosmid 85 (tetR version)
Select for resistance to oxytetracycline
Select for resistance to galactose
L
K
epoF
epoE
pKOS90-22 (linear)
Upstream region
Cosmid 1A2 (tetR version)
epoD
epoC
epoB
epoA

FIGURE 3

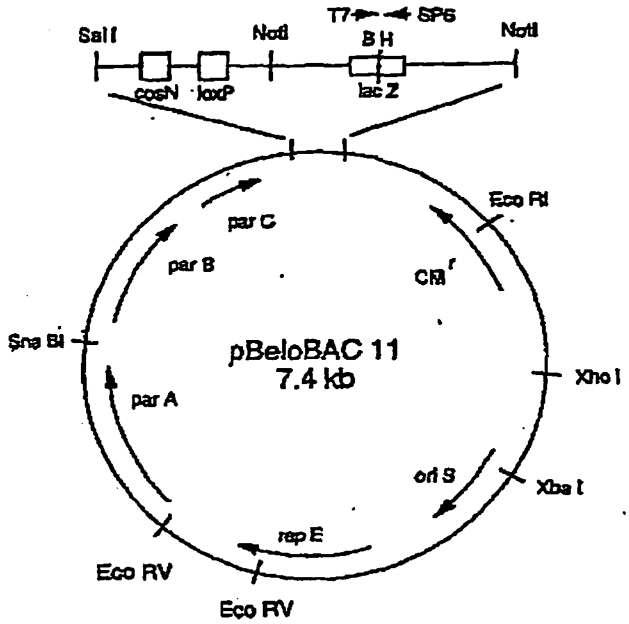
SalI
NotI
T7 SP6
B H
NotI
cosN
loxP
lac Z
Eco RI
par C
par B
CM^r
Sna BI
pBeloBAC 11
7.4 kb
Xho I
par A
ori S
Xba I
rep E
Eco RV
Eco RV

FIGURE 4

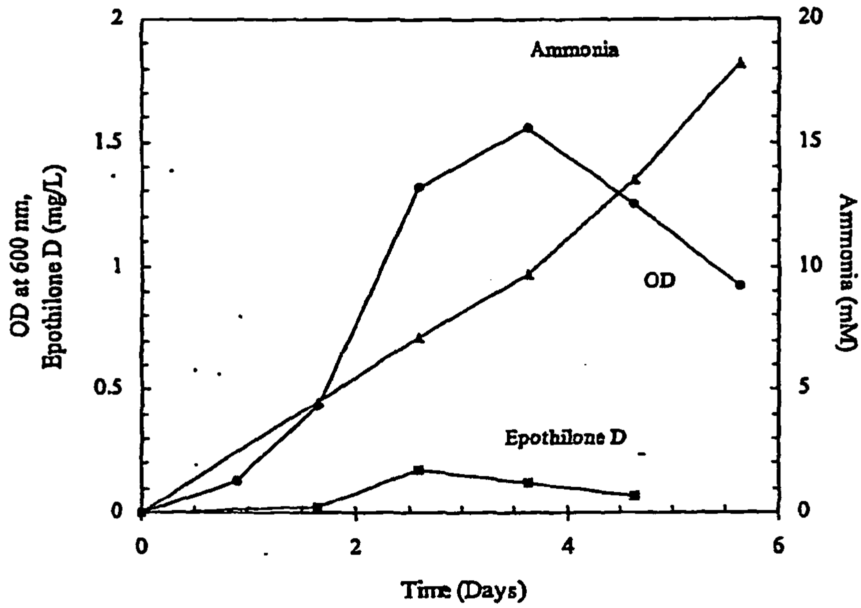
2
1.5
1
0.5
0
20
15
10
5
0
Ammonia
OD
Epothilone D
OD at 600 nm,
Epothilone D (mg/L)
Ammonia (mM)
0
2
4
6
Time (Days)

FIGURE 5

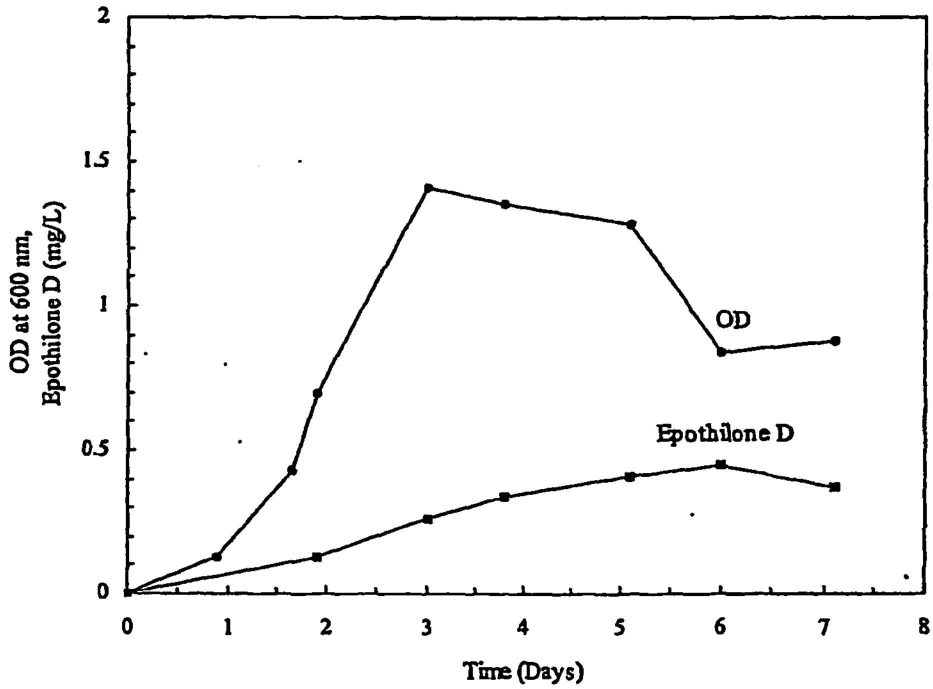
2
1.5
1
0.5
0
OD at 600 nm,
Epothilone D (mg/L)
OD
Epothilone D
0
1
2
3
4
5
6
7
8
Time (Days)

**FIGURE 6**

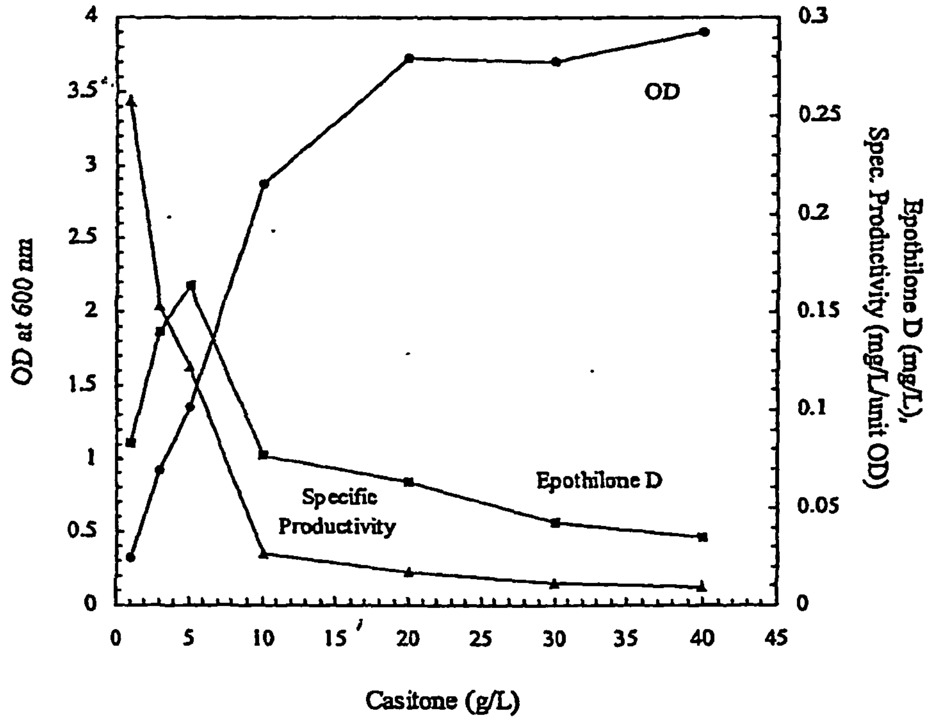

4
3.5
3
2.5
2
1.5
1
0.5
0
OD at 600 nm
0.3
0.25
0.2
0.15
0.1
0.05
0
Epothilone D (mg/L),
Spec. Productivity (mg/L/unit OD)
OD
Epothilone D
Specific
Productivity
0
5
10
15
20
25
30
35
40
45
Casitone (g/L)


FIGURE 7

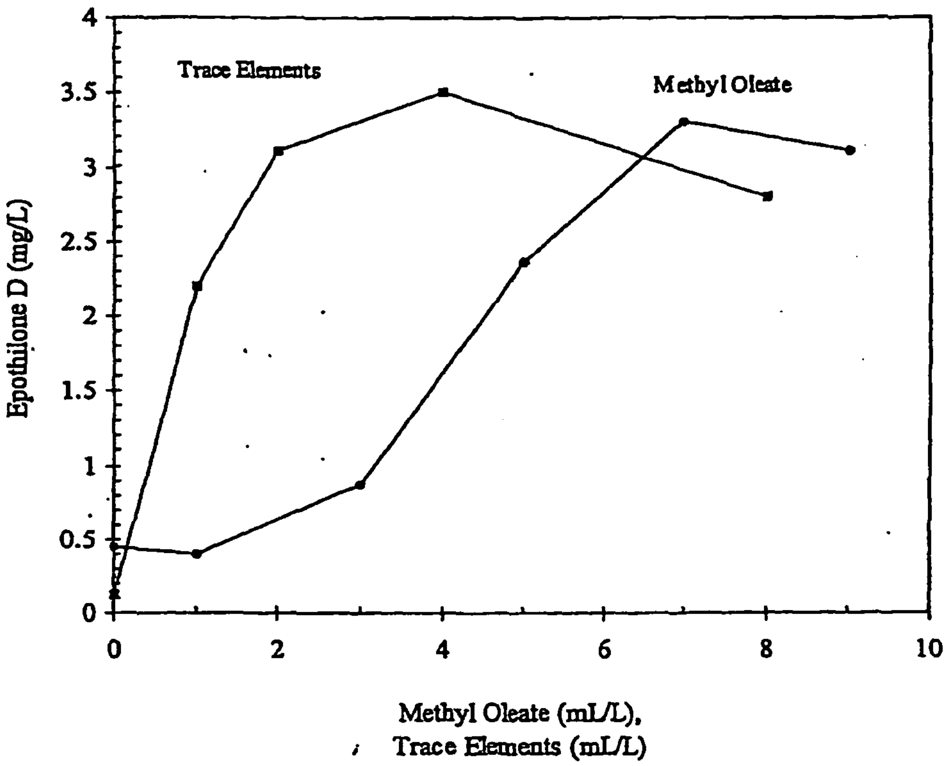
Trace Elements
Methyl Oleate
Epothilone D (mg/L)
4
3.5
3
2.5
2
1.5
1
0.5
0
0
2
4
6
8
10
Methyl Oleate (mL/L),
Trace Elements (mL/L)

FIGURE 8

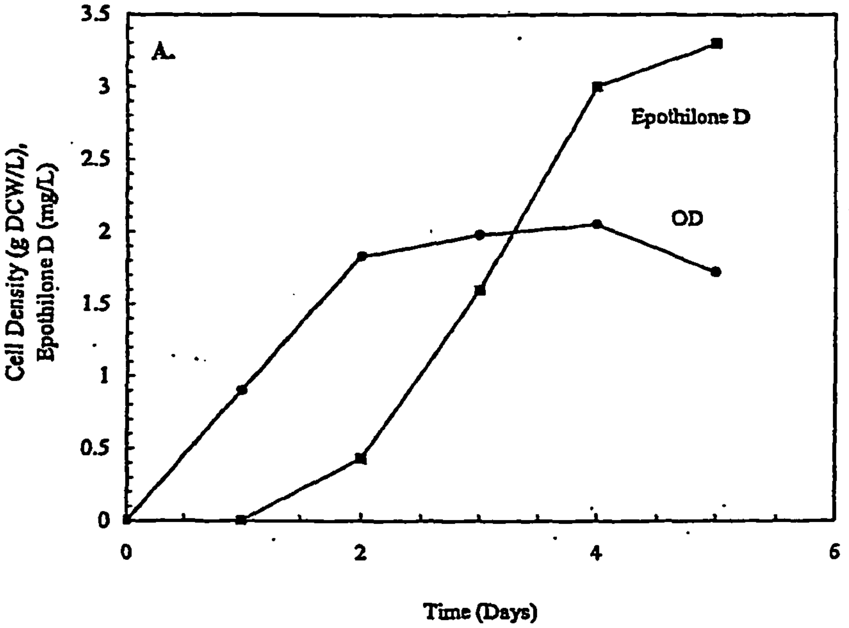
A.
Epothilone D
OD
Cell Density (g DCW/L), Epothilone D (mg/L)
Time (Days)
0
0.5
1
1.5
2
2.5
3
3.5
0
2
4
6

FIGURE 9A

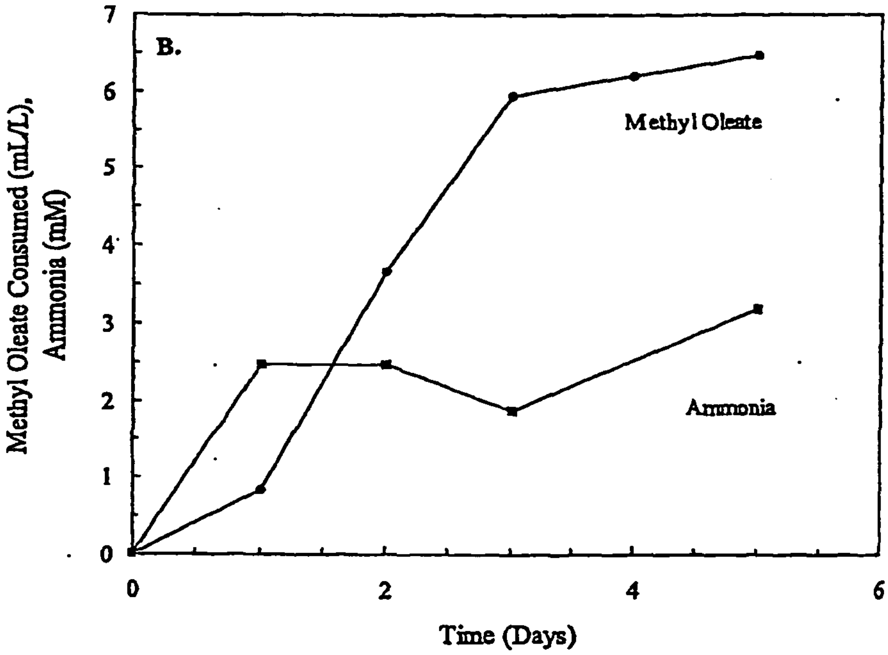
B.
Methyl Oleate
Ammonia
Methyl Oleate Consumed (mL/L), Ammonia (mM)
Time (Days)
0
1
2
3
4
5
6
7
0
2
4
6

FIGURE 9B

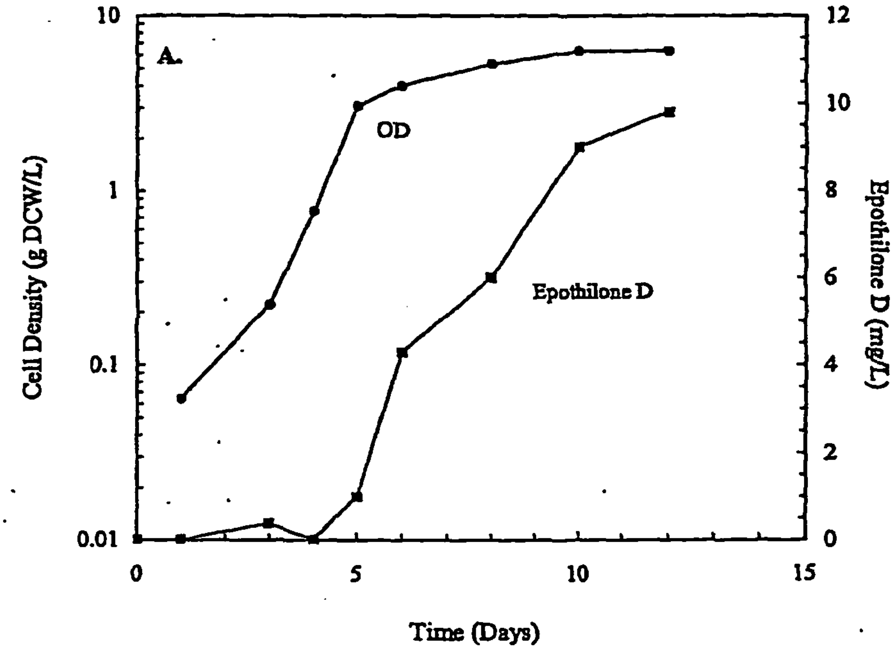
A.
OD
Epothilone D
Cell Density (g DCW/L)
Epothilone D (mg/L)
Time (Days)
10
1
0.1
0.01
12
10
8
6
4
2
0
0
5
10
15

FIGURE 10A

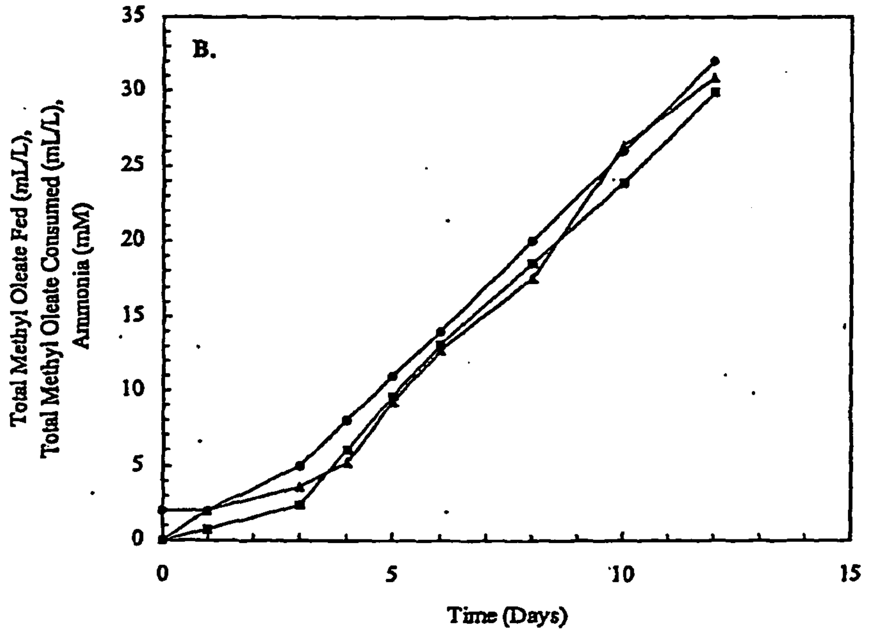
B.
35
30
25
20
15
10
5
0
0
5
10
15
Total Methyl Oleate Fed (mL/L),
Total Methyl Oleate Consumed (mL/L),
Ammonia (mM)
Time (Days)

FIGURE 10B

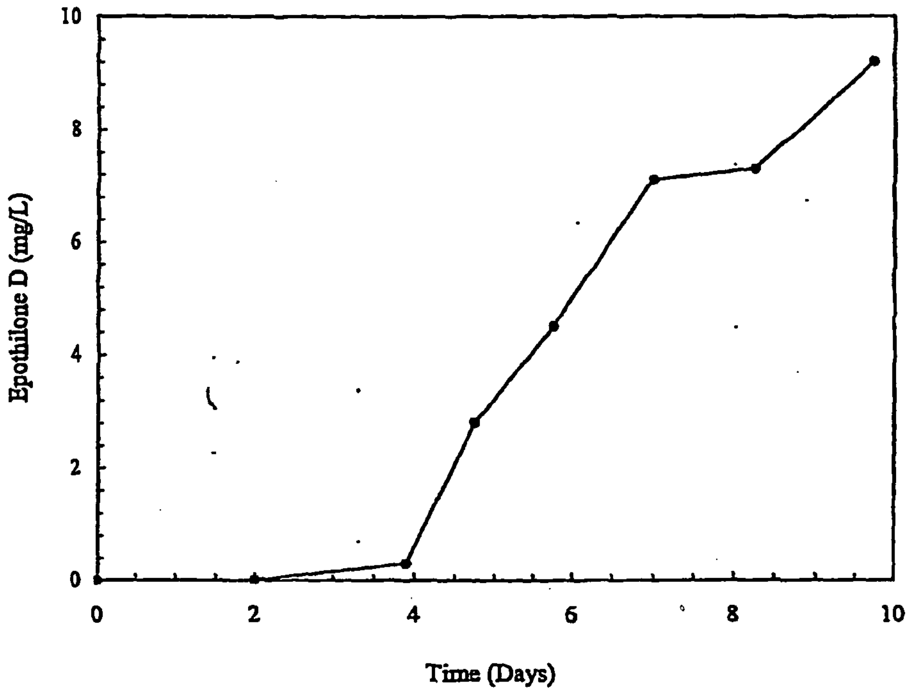


FIGURE 11

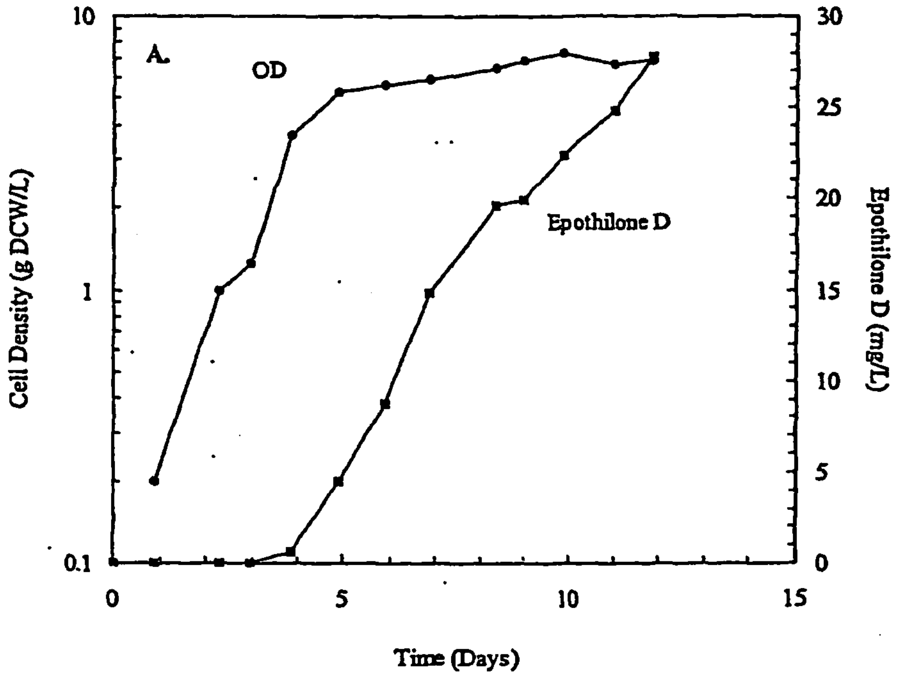
A.
OD
Epothilone D
10
1
0.1
30
25
20
15
10
5
0
0
5
10
15
Cell Density (g DCW/L)
Epothilone D (mg/L)
Time (Days)

**FIGURE 12A**

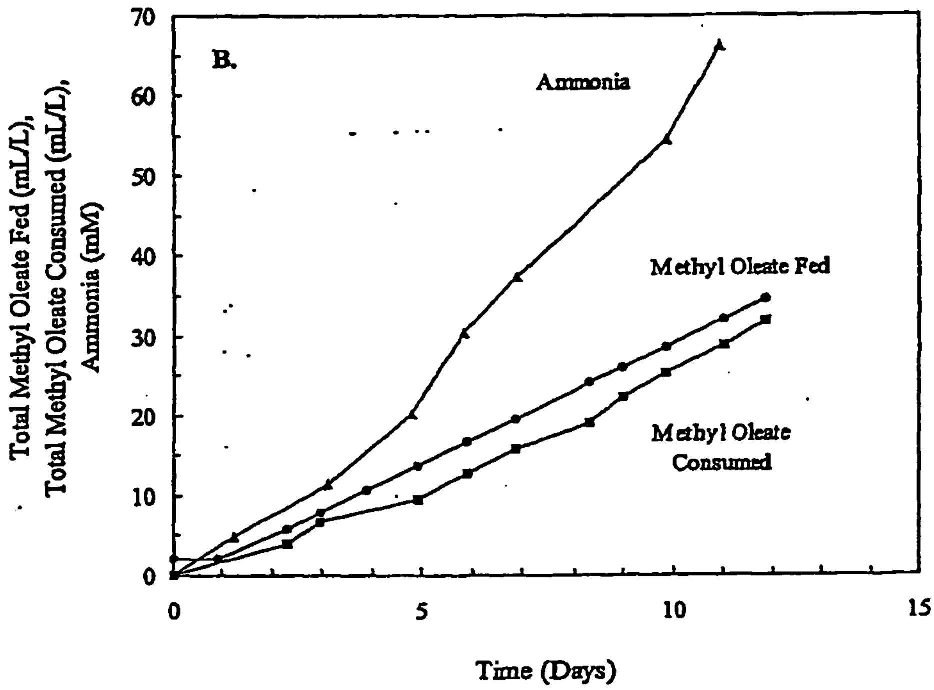
B.
Ammonia
Methyl Oleate Fed
Methyl Oleate
Consumed
Total Methyl Oleate Fed (mL/L),
Total Methyl Oleate Consumed (mL/L),
Ammonia (mM)
70
60
50
40
30
20
10
0
0
5
10
15
Time (Days)

FIGURE 12B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5962290 A
- US 5672491 A
- US 5712146 A
- US 5830750 A
- US 5843718 A
- US 9849315 W
- US 9702358 W
- US 6033883 A
- US 9713845 W
- US 00031247 W
- DE 4138042
- US 9965913 W
- US 0023452 W
- US 0000485 W
- US 9967253 W
- US 9967252 W
- US 9954330 W
- US 9954319 W
- US 9954318 W
- US 9943653 W
- US 9943320 W
- US 9942602 W
- US 9940047 W
- US 9927890 W
- US 9907692 W
- US 9902514 W
- US 9901124 W
- US 9825929 W
- US 9822461 W
- US 9808849 W
- US 9719086 W
- US 5969145 A
- US 9827203 W
- US 00063361 W
- US 00031237 W
- US 00039276 W
- US 9310121 W
- US 9939694 W


### Non-patent literature cited in the description


- **Tang et al.** Clowning and heterologous expression of the epothilone gene cluster. *Science,* 28 January 2000, vol. 287, 640-642
- **Gerth et al.** *J. Antibiotics,* 1996, vol. 49, 560-563
- **Bollag et al.** *Cancer Res.,* 1995, vol. 55, 2325-2333
- **Hofle et al.** Epothilone A and B - novel 16-membered macrolides with cytotoxic activity: isolation, crystal structure, and conformation in solution. *Angew. Crem. Int. Ed. Engl.,* 1996, vol. 35 (13/14), 1567-1569
- **Balog et al.** Total synthesis of (-)-epothilone A. *Angew. Chem. Int. Ed. Engl.,* 1996, vol. 35 (23/24), 2801-2803
- **Su et al.** Total synthesis of (-)-epothilone B: an extension of the Suzuki coupling method and insights into structure-activity relationships of the epothilones. *Angew. Chem. Int. Ed. Engl.,* 1997, vol. 36 (7), 757-759
- **Meng et al.** Total syntheses of epothilones A and B. *JACS,* 1997, vol. 119 (42), 10073-10092
- **Balog et al.** A novel aldol condensation with 2-methyl-4-pentenal and its application to an improved total synthesis of epothilone B. *Angew. Chem. Int. Ed. Engl.,* 1998, vol. 37 (19), 2675-2678
- **Su et al.** Structure-activity relationships of the epothilones and the first in vivo comparison with paclitaxel. *Angew. Chem. Int. Ed. Engl.,* 1997, vol. 36 (19), 2093-2096
- **Chou et al.** Desoxyepothilone B: an efficacious microtubule-targeted antitumor agent with a promising in vivo profile relative to epothilone B. *Proc. Natl. Acad. Sci. USA,* August 1998, vol. 95, 9642-9647
- **T. H. Greene ; P.G. M. Wuts.** Protective Groups in Organic Synthesis. John Wiley & Sons, 1999
- **Campos et al.** *J. Mol. Biol.,* 1978, vol. 119, 167-178
- **Hodgkin ; Kaiser.** *Mol. Gen. Genet.,* 1979, vol. 171, 177-191
- **Kaiser.** *Proc. Natl. Acad. Sci. USA,* 1979, vol. 76, 5952-5956
- **Wu ; Kaiser.** Regulation of expression of the pilA gene in Myxococcus xanthus. *J. Bact.,* December 1997, vol. 179 (24), 7748-7758
- **Jaoua et al.** *Plasmid,* 1992, vol. 28, 157-165
- **Salmi et al.** *J. Bact.,* February 1998, vol. 180 (3), 614-621

- **Campos ; Zusman.** Regulation of development in Myxococcus xanthus: effect of 3': 5'-cyclic AMP, ADP, and nutrition. *Proc. Natl. Acad. Sci. USA,* 1975, vol. 72, 518-522
- **Ueki et al.** *Gene,* 1996, vol. 183, 153-157
- **Strong et al.** *Nucleic Acids Res.,* 1997, vol. 19, 3959-3961
- **Sheng et al.** *Nucleic Acids Res.,* 1995, vol. 23, 1990-1996
- **Kafeshi et al.** *Mol. Microbiol.,* 1995, vol. 15, 483-494
- **Hamilton et al.** *J. Bact.,* 1989, vol. 171 (9), 4617-4622
- **Link et al.** *J. Bact.,* October 1997, vol. 179 (20), 6228-6237
- **Magrini et al.** *J. Bact.,* July 1999, vol. 181 (13), 4062-4070
- **Bollag et al.** Epothilones, a new class of microtubule-stabilizing agents with a taxol-like mechanism of action. *Cancer Res,* 1995, vol. 55, 2325-2333
- **Choi et al.** Effects of rapeseed oil on activity of methylmalonyl-CoA carboxyltransferase in culture of Streptomyces fradiae. *Biosci Biotechnol Biochem,* 1998, 62902-62906
- **Chou et al.** Desoxyepothilone B: An efficacious miczotubule-targeted antitumor agent with a promising in vivo profile relative to epothilone B. *Proc Natl Acad Sci USA,* 1998, vol. 95, 9642-9647
- **Gerth et al.** Epothilons A and B: Antifungal and cytotoxic compounds from Sorangium cellulosum (myxobacteria) - production, physico-chemical, an biological properties. *J Antibiot (Tokyo),* 1996, vol. 49, 560-563
- **Gerth et al.** Studies on the biosynthesis of epothilones: the biosynthetic origin of the carbon skeleton. *J Antibiot (Tokyo),* 2000, vol. 53, 1373-1377
- **Gerth et al.** Studies on the biosynthesis of epothilones: The PKS and epothilone C/D monooxygenase. *J Antibiot (Tokyo),* 2001, vol. 54, 144-148
- **Harris et al.** New chemical synthesis of the promising cancer chemotherapeutic agent 12,13-desoxyepothilone B: Discovery of a surprising long-range effect on the diastereoselectivity of an aldol condensation. *J Am Chem Soc,* 1999, vol. 12, 7050-7062
- **Hecht et al.** Hollow fiber supported gas membrane for in situ removal of ammonium during an antibiotic fermentation. *Biotechnol Bioeng,* 1990, vol. 35, 1042-1050
- **Junker et al.** Use of soybean oil and ammonium sulfate additions to optimize secondary metabolite production. *Biotechnol Bioeng,* 1998, vol. 60, 580-588
- **Kowalski et al.** Activities of the microtubule-stabilizing agents epothilones A and B with purified tubulin and in cells resistant to paclitaxel. *J Biol Chem,* 1997, vol. 272, 2534-41
- **Meng et al.** Remote effects in macrolide formation through ring-forming olefin metathesis: An application to the synthesis of fully active epothilone congeners. *J Am Chem Soc,* 1997, vol. 119, 2733-2734
- **Mirjalili et al.** The effect of rapeseed oil uptake on the production of erythromycin and triketide lactone by Saccharopolyspora erythraea. *Biotechnol Prog,* 1999, vol. 15, 911-918
- **Molnár et al.** The biosynthetic gene cluster for the microtubule-stabilizing agents epothilones A and B from Sorangium cellulosum So ce90. *Chem Biol,* 2000, vol. 7, 97-109
- **Reichenbach et al.** The Prokaryotes II. Springer-Verlag, 3417-3487
- Production of Bioactive Secondary Metabolites. **Reichenbach et al.** Myxobacteria II. American Society for Microbiology, 1993, 347-397
- **Sinha et al.** The antibody catalysis route to the total synthesis of epothilones. *Proc Natl Acad Sci USA,* 1998, vol. 95, 14603-14608
- **Su et al.** Structure-activity relationships of the epothilones and the first in vivo comparison with paclitaxel. *Angew Chem Int Ed Engl,* 1997, vol. 36, 2093-2096
- **Tang et al.** Cloning and heterologous expression of the epothilone gene cluster. *Science,* 2000, vol. 287, 640-642